(19) Europäisches Patentamt
European Patent Office
Office européen des brevets

(11) Veröffentlichungsnummer: **0 395 578 A2**

(12) # EUROPÄISCHE PATENTANMELDUNG

(21) Anmeldenummer: **90810303.9**

(22) Anmeldetag: **18.04.90**

(51) Int. Cl.5: **C07C 271/62, C07C 271/20, A01N 47/10**

(30) Priorität: **24.04.89 CH 1557/89**

(43) Veröffentlichungstag der Anmeldung: **31.10.90 Patentblatt 90/44**

(84) Benannte Vertragsstaaten: **BE CH DE DK ES FR GB IT LI NL**

(71) Anmelder: **CIBA-GEIGY AG**
**Klybeckstrasse 141**
**CH-4002 Basel(CH)**

(72) Erfinder: **Karrer, Friedrich, Dr.**
**Rebbergstrasse 5**
**CH-4800 Zofingen(CH)**

(54) **Acylierte Carbaminsäureester.**

(57) Neue N-acylierte 2-[4-(4-Fluorphenoxy)-phenoxy]-äthylcarbaminsäureester der Formel I

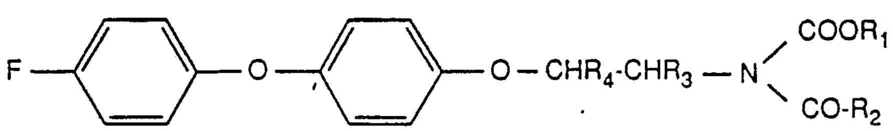

(I),

worin
$R_1$ für $C_1$-$C_8$-Alkyl, $C_3$-$C_4$-Alkenyl oder $C_3$-$C_4$-Alkinyl,
$R_2$ für $C_1$-$C_8$-Alkyl, $C_1$-$C_8$-Alkoxy, -CO-$R_5$ oder -$NR_6R_7$,
$R_3$ und $R_4$ unabhängig voneinander für Wasserstoff oder Methyl,
$R_5$ für $C_1$-$C_8$-Alkoxy oder -$NR_8R_9$,
$R_6$ für $C_1$-$C_4$-Alkyl,
$R_7$ für $C_1$-$C_4$-Alkyl oder
$R_6$ und $R_7$ zusammen für eine $C_4$-$C_6$-Alkylenkette, welche durch Sauerstoff, Schwefel oder -$NCH_3$- unterbrochen sein kann,
$R_8$ für Wasserstoff oder $C_1$-$C_4$-Alkyl und
$R_9$ für Wasserstoff, $C_1$-$C_4$-Alkyl, Benzyl, Phenyl oder durch Halogen oder Methyl substituiertes Phenyl oder
$R_8$ und $R_9$ zusammen für eine $C_4$-$C_6$-Alkylenkette, welche durch Sauerstoff, Schwefel oder -$NCH_3$- unterbrochen sein kann, stehen.

EP 0 395 578 A2

# Acylierte Carbaminsäureester

Die vorliegende Erfindung betrifft neue N-acylierte 2-[4-(4-Fluorphenoxy)-phenoxy]-äthylcarbaminsäureester, ihre Herstellung, ihre Verwendung in der Schädlingsbekämpfung sowie Schädlingsbekämpfungsmittel, welche diese Carbaminsäureester als Wirkstoff enthalten.

Die erfindungsgemässen N-Acyl-2-[4-(4-fluorphenoxy)-phenoxy]-äthylcarbaminsäureester entsprechen der Formel I

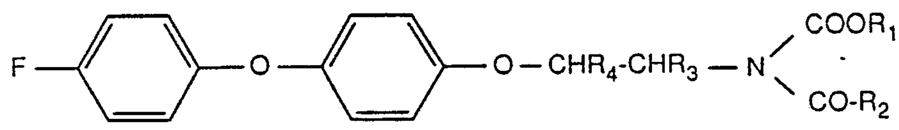

(I),

worin

$R_1$ für $C_1$-$C_8$-Alkyl, $C_3$-$C_4$-Alkenyl oder $C_3$-$C_4$-Alkinyl,
$R_2$ für $C_1$-$C_8$-Alkyl, $C_1$-$C_8$-Alkoxy, -CO-$R_5$ oder -$NR_6R_7$,
$R_3$ und $R_4$ unabhängig voneinander für Wasserstoff oder Methyl,
$R_5$ für $C_1$-$C_8$-Alkoxy oder -$NR_8R_9$,
$R_6$ für $C_1$-$C_4$-Alkyl,
$R_7$ für $C_1$-$C_4$-Alkyl oder
$R_6$ und $R_7$ zusammen für eine $C_4$-$C_6$-Alkylenkette, welche durch Sauerstoff, Schwefel oder -$NCH_3$- unterbrochen sein kann,
$R_8$ für Wasserstoff oder $C_1$-$C_4$-Alkyl und
$R_9$ für Wasserstoff, $C_1$-$C_4$-Alkyl, Benzyl, Phenyl oder durch Halogen oder Methyl substituiertes Phenyl oder
$R_8$ und $R_9$ zusammen für eine $C_4$-$C_6$-Alkylenkette, welche durch Sauerstoff, Schwefel oder -$NCH_3$- unterbrochen sein kann, stehen.

Unter Halogen sind in der Definition von $R_9$ Fluor, Chlor, Brom oder Jod zu verstehen, vorzugsweise aber Chlor.

$C_1$-$C_8$-Alkylgruppen können geradkettig oder verzweigt sein. Als Beispiele solcher Reste seien Methyl, Aethyl, Propyl, Isopropyl oder Butyl und seine Isomeren sowie die möglichen Strukturisomeren der $C_5$-$C_8$-Alkyle genannt. Bevorzugte Alkylgruppen enthalten höchstens 4 Kohlenstoffatome. Besonders bevorzugt sind Methyl und Aethyl.

$C_1$-$C_8$-Alkoxyreste der Definition von $R_2$ und $R_5$ sind im Rahmen vorliegender Erfindung Methoxy, Aethoxy, Propoxy, Isopropoxy oder die vier isomeren Butoxy oder die möglichen Strukturisomeren der $C_5$-$C_8$-Alkoxyreste. Hervorgehoben werden die kurzkettigen Alkoxygruppen mit weniger als 5 Kohlenstoffatomen. Bevorzugt sind hiervon Methoxy und Aethoxy.

Falls $R_6$ und $R_7$, oder $R_8$ und $R_9$ gemeinsam eine gegebenenfalls durch Sauerstoff, Schwefel oder -$NCH_3$- unterbrochene $C_4$-$C_6$-Alkylenkette bedeuten, wird von den Gruppen -$NR_6R_7$ oder -$NR_8R_9$ ein über das Stickstoffatom gebundener Heterocyclus gebildet. Diesen Heterocyclen liegen die Strukturen beispielsweise von Pyrrolidin, Piperidin, Perhydroazepin, Oxazolidin, Thiazolidin, Imidazolidin, Pyrazolidin, Perhydropyrimidin, Morpholin, Thiomorpholin, Perhydropyridazin, Isoxazolidin, Isothiazolidin oder Piperazin zugrunde.

Pestizide Aethylcarbaminsäure-Derivate sind aus der Literatur schon verschiedentlich bekannt geworden, jedoch befriedigen diese Substanzen bezüglich des erzielten Wirkungsspektrums nicht voll oder nur teilweise. Solche Verbindungen sind beispielsweise bekannt aus den US-Patenten 4 080 470, 4 215 139, 4 413 010, 4 555 405, 4 608 389 und 4 745 128 sowie den Deutschen Offenlegungsschriften DE-OS 3 320 534 und 3 334 983. Es besteht somit noch immer ein Bedürfnis nach Wirkstoffen dieser Substanzklasse mit verbesserten Eigenschaften.

Es wurde nun gefunden, dass die erfindungsgemässen Verbindungen der Formel I bei günstiger Warmblüter-, Fisch- und Pflanzenverträglichkeit wertvolle Wirkstoffe in der Schädlingsbekämpfung sind. Insbesondere betrifft die Anwendung der erfindungsgemässen Wirkstoffe Insekten und Spinnentiere, die in Nutz- und Zierpflanzen in der Landwirtschaft, insbesondere in Baumwoll-, Gemüse- und Obstpflanzungen, im Forst, Im Vorrats- und Materialschutz sowie im Hygienesektor insbesondere an Haus- und Nutztieren vorkommen. Sie sind gegen alle oder einzelne Entwicklungsstadien von normal sensiblen aber auch resistenten Arten wirksam. Dabei kann sich ihre Wirkung in unmittelbarer Abtötung der Schädlinge oder erst nach einiger Zeit, beispielsweise bei einer Häutung, oder in einer verminderten Eiablage und/oder Schlupfrate zeigen. Zu den oben erwähnten Schädlingen gehören:

aus der Ordnung Lepidoptera zum Beispiel Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.; aus der Ordnung Coleoptera zum Beispiel Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp.·Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.; aus der Ordnung der Orthoptera zum Beispiel Blatta spp., Blattella spp., Gryllotalpa spp., Leucophaea maderae, Locusta spp., Periplaneta spp. und Schistocerca spp.; aus der Ordnung der Isoptera zum Beispiel Reticulitermes spp.; aus der Ordnung der Psocoptera zum Beispiel Liposcelis spp.;
aus der Ordnung der Anoplura zum Beispiel Haematopinus spp., Linognathus spp. Pediculus spp., Pemphigus spp. und Phylloxera spp.; aus der Ordnung der Mallophaga zum Beispiel Damalinea spp. und Trichodectes spp.; aus der Ordnung der Thysanoptera zum Beispiel Frankliniella spp., Hercinothrips spp., Taeniothrips spp., Thrips palmi, Thrips tabaci und Scirtothrips aurantii; aus der Ordnung der Heteroptera zum Beispiel Cimex spp., Distantiella theobroma, Dysdercus spp., Euchistus spp. Eurygaster spp. Leptocorisa spp., Nezara spp., Piesma spp., Rhodnius spp., Sahlbergella singularis, Scotinophara spp. und Triatoma spp.; aus der Ordnung der Homoptera zum Beispiel Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri; aus der Ordnung der Hymenoptera zum Beispiel Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.; aus der Ordnung der Diptera zum Beispiel Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp. Oscinella frit, Pegomyia hyoscyami, Phorbia spp., Rhagoletis pomonella, Sciara spp., Stomoxys spp., Tabanus spp., Tannia spp. und Tipula spp.; aus der Ordnung der Siphonaptera z.B. Ceratophyllus spp., Xenopsylla cheopis,
aus der Ordnung der Acarina zum Beispiel Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. und Tetranychus spp.; und aus der Ordnung der Thysanura zum Beispiel Lepisma saccharina.

Von besonderer Bedeutung hat sich bei den erfindungsgemässen Verbindungen die Verwendung bei der Bekämpfung von Reiszikaden zum Beispiel der Familien Delphacidae und Cicadellidae wie Nilaparvata lugens, Laodelphax striatellus und Nephotettix cincticeps erwiesen. Ebenfalls herausragende Wirkung zeigen die Wirkstoffe der Formel I gegen die schwierig unter Kontrolle zu bringenden sogenannten "Weissen Fliegen" der Familie Aleyrodidae, Gattungen Bemisia und Trialeurodes wie Bemisia tabaci oder Trialeurodes vaporarium. Sehr gute Wirkungen erzielen die Verbindungen der Formel I gegen Obstbaumschädlinge der Familien Tortricidae und Olethreutidae, z.B. der Gattungen Cydia, Adoxophyes und Lobesia, mit den Arten cydia pomonella, Adoxophyes orana und Lobesia botrana. Bei der Bekämpfung von tierparasitären Schädlingen, insbesondere an Haus- und Nutztieren, kommen vor allem Ektoparasiten wie zum Beispiel Milben und Zecken, wie Boophilus microplus und Dermanyssus gallinae, Dipteren wie Lucilia sericata und Flöhe wie zum Beispiel Ctenocephalides felis in Betracht.

Im wesentlichen bewirken die Verbindungen der Formel I in den Zielgruppen von Schädlingen eine Wachstumshemmung oder Entwicklungshemmung bei den verschiedenen Entwicklungsstufen, so dass die Verminderung des Schädlingsbefalls auf Entwicklungsstörungen der Schädlinge, insbesondere einen chemosterilisierenden und oviziden Effekt zurückzuführen ist.

Wegen ihrer vorteilhaften Wirkung sind unter den Verbindungen der Formel I solche hervorzuheben, worin $R_1$ für $C_1$-$C_1$-Alkyl, $R_2$ für $C_1$-$C_4$-Alkyl, $C_1$-$C_4$-Alkoxy, $C_1$-$C_4$-Alkoxycarbonyl, Di-($C_1$-$C_4$-alkyl)-amino oder -$CONR_8R_9$ stehen, wobei $R_8$ Wasserstoff oder $C_1$-$C_4$-Alkyl, $R_9$ Wasserstoff, $C_1$-$C_4$-Alkyl oder Phenyl oder $R_8$ und $R_9$ zusammen eine $C_4$-$C_6$-Alkylenkette, die durch Sauerstoff unterbrochen sein kann, bedeuten.

Aus dieser Gruppe von bevorzugten Verbindungen sind wiederum solche bevorzugt, worin $R_2$ für $C_1$-$C_4$-Alkyl, $C_1$-$C_4$-Alkoxy, $C_1$-$C_4$-Alkoxycarbonyl, Di-($C_1$-$C_4$-alkyl)-aminocarbonyl oder $C_4$-$C_6$-Alkylenaminocarbonyl steht.

Eine besonders bevorzugte Gruppe von Verbindungen der Formel I ist dadurch gekennzeichnet, dass $R_1$ für $C_1$-$C_4$-Alkyl, $R_2$ für $C_1$-$C_4$-Alkyl, $C_1$-$C_4$-Alkoxy, Di-($C_1$-$C_2$-alkyl)-aminocarbonyl oder $C_1$-$C_4$-Alkoxycarbonyl, $R_3$ für Methyl und $R_4$ für Wasserstoff steht.

Als bevorzugte Einzelverbindungen gemäss vorliegender Erfindung sind zu nennen:

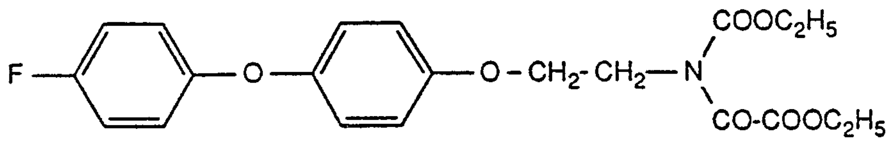

,

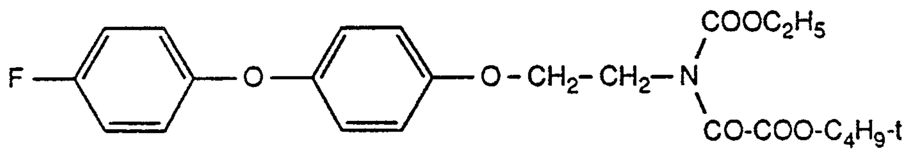

,

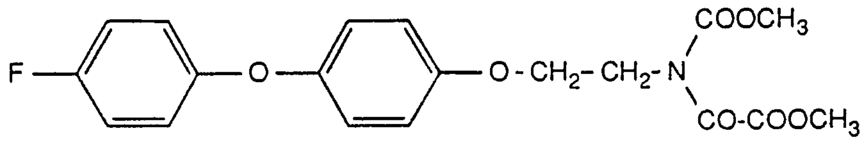
,

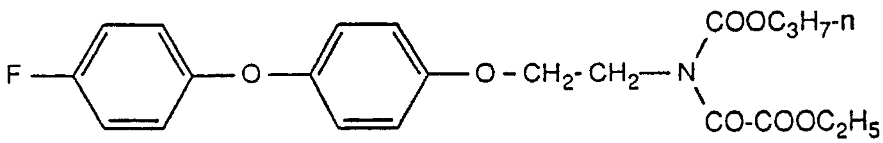
,

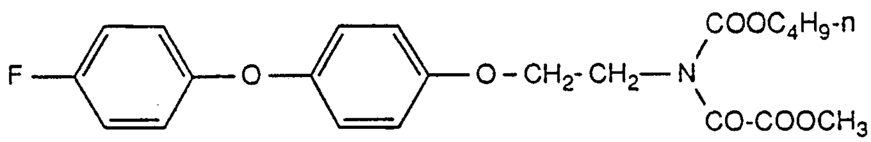
,

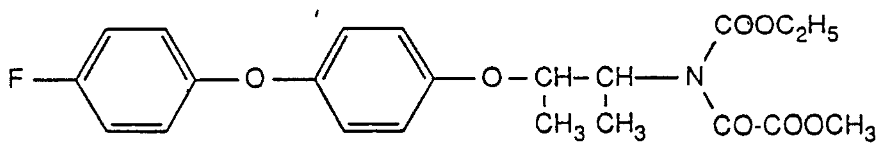
,

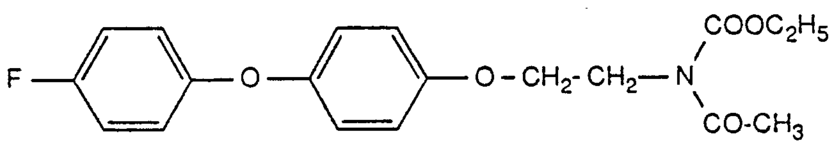
,

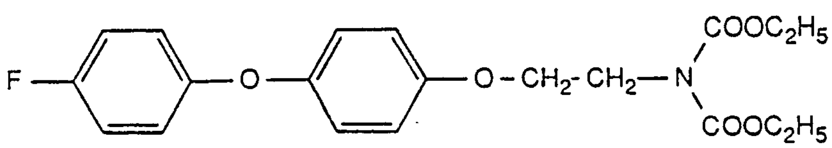
,

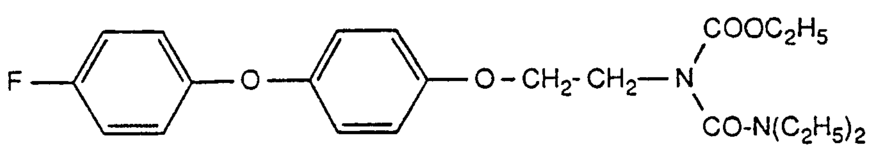
,

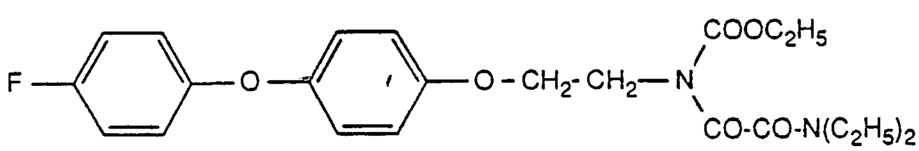
,

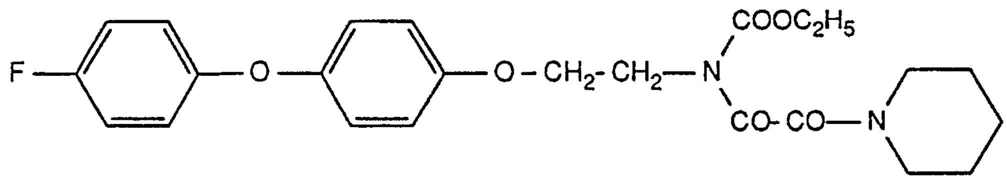
,

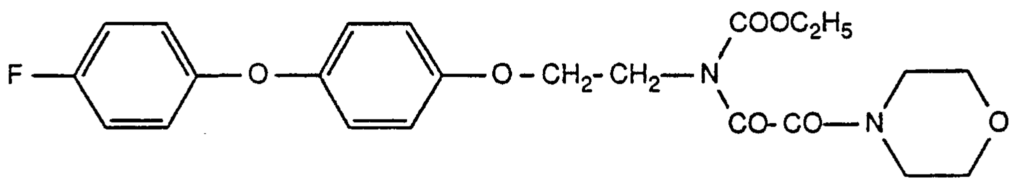
,

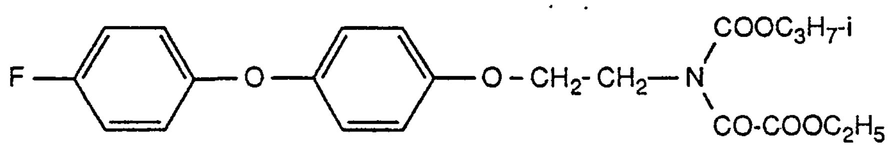
,

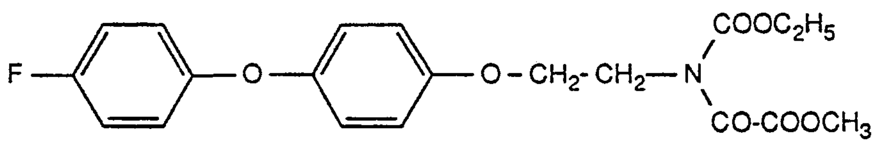
und

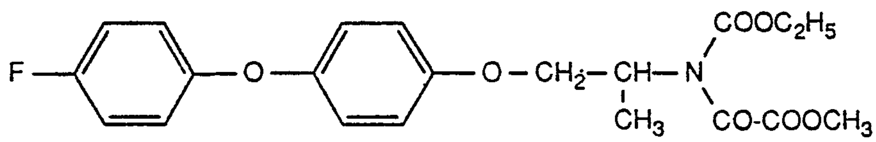
.

Die erfindungsgemässen Verbindungen der Formel I können nach an sich bekannten Methoden hergestellt werden. So kann man die Verbindungen der Formel I beispielsweise erhalten, indem man einen Fluorphenoxyphenoxyäthylcarbaminsäureester der Formel II

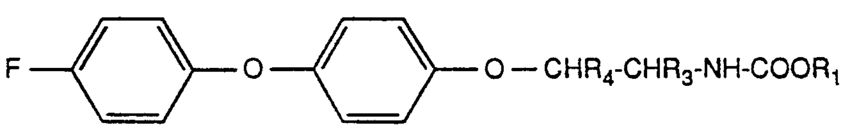
(II)

worin $R_1$, $R_3$ und $R_4$ die unter Formel I angegebenen Bedeutungen haben, mit einer aktivierten Acylverbindung der Formel III

$X$-$CO$-$R_2$ (III)

worin $R_2$ die unter Formel I angegebene Bedeutung hat und X für Chlor, Brom, -O-CO-$R_2$ oder -O-CO($C_1$-$C_4$-Alkyl) steht, acyliert.

Das erfindungsgemässe Herstellungsverfahren wird vorzugsweise in Gegenwart einer Base durchgeführt. Als Basen eignen sich sowohl anorganische Basen, wie Alkali- und Erdalkalicarbonate oder -hydrogencarbonate, beispielsweise Natriumcarbonat, Kaliumcarbonat, Natriumhydrogencarbonat oder Kaliumhydrogencarbonat, oder Alkali-und Erdalkalihydride, wie Natriumhydrid oder Calciumhydrid; als auch organische Basen, wie tertiäre Amine, beispielsweise Trialkylamine, wie Triäthylamin, Diisopropyläthylamin, Pyridin, Dimethylaminopyridin, 1,4-Diazabicyclo[2,2,2]octan oder 1,8-Diazabicyclo[5,4,0]undec-7en, Alkalialkoholate, wie Natriummethylat, Natriumäthylat oder Kalium-tert.butylat, oder Alkalialkylverbindungen, wie Butyllithium.

Mit Vorteil werden die Umsetzungen zur Herstellung der erfindungsgemässen Verbindungen der Formel I in inerten, aprotischen organischen Lösungsmitteln durchgeführt. Solche Lösungsmittel sind Kohlenwasserstoffe, wie Hexan, Heptan, Cyclohexan, Ligroin, Benzol, Toluol, Xylol, Mesitylen oder Tetralin; chlorierte Kohlenwasserstoffe, wie Dichlormethan, Chloroform, Tetrachlorkohlenstoff, Chlorbenzol, Dichlorbenzol, Trichloräthan oder Tetrachloräthan; Aether wie Diäthyläther, 1,2-Dimethoxyäther, Tetrahydrofuran oder Dioxan; Nitrile, wie Acetonitril oder Propionitril; Dimethylsulfoxid oder Sulfolan; oder Dialkylformamide, wie Dimethylformamid oder Dimethylacetamid.

Die Reaktionstemperaturen des erfindungsgemässen Verfahrens liegen je nach Wahl des Lösungsmittels und der fakultativ zu verwendenden Base im allgemeinen zwischen -10° C und dem Siedepunkt des Reaktionsgemisches, meist zwischen 0° und +130° C. Bevorzugte Reaktionstemperaturen liegen zwischen +20° C und +100° C. Bei Verwendung sehr reaktiver Reagenzien, wie beispielsweise Butyllithium wird die Reaktionstemperatur vorzugsweise beträchtlich tiefer, etwa zwischen -80° C und +20° C, gehalten. Bevorzugt ist hier zum Beispiel der Bereich zwischen -70° C und 0° C.

Neben dem genannten erfindungsgemässen Verfahren zur Herstellung der Verbindungen der Formel I durch Umsetzen von II mit III, hat es sich bei der Herstellung der Verbindungen der Formel I, worin $R_2$ für die Gruppe -CO-$R_5$ steht, als vorteilhaft erwiesen, wenn man die Verbindung der Formel II zunächst in einem inerten Lösungsmittel mit Oxalylchlorid umsetzt, und das entstehende Zwischenprodukt der Formel

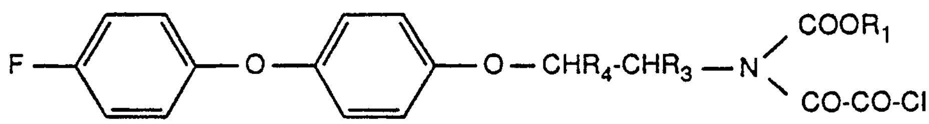

worin $R_1$, $R_3$ und $R_4$ die unter Formel I gegebenen Bedeutungen haben, in Gegenwart einer Base mit einem Alkohol oder einem Amin der Formel

H-$R_5$

worin $R_5$ die unter Formel I gegebene Bedeutung hat, umsetzt.

Die Ausgangsmaterialien der Formeln II und III sind zum grossen Teil bekannt. Neue Einzelverbindungen, welche unter die Formeln II und III fallen, können nach bekannten Methoden hergestellt werden. So erhält man die Ausgangsprodukte der Formel II auf einfachem Wege durch Umsetzung eines 2-[4-(4-Fluorphenoxy)-phenoxy]-äthylamins der Formel IV

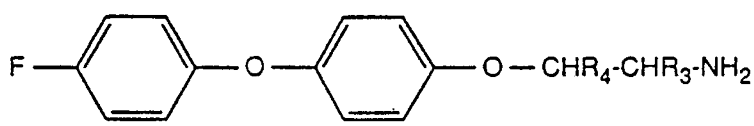

(IV)

worin $R_3$ und $R_4$ die unter Formel I gegebenen Bedeutungen haben, mit einem Halogenameisensäureester der Formel V

Hal-CO-O$R_1$ (V)

worin $R_1$ die unter Formel I gegebene Bedeutung hat und Hal für Halogen, vorzugsweise Brom oder Chlor steht, in Gegenwart einer Base.

Die Verbindungen der Formel II sind auch erhältlich durch Umsetzung von 4-(4-Fluorphenoxy)-phenol mit einem 2-Halogenäthyl-carbaminsäureester X-$CHR_4$-$CHR_3$-NH-$COOR_1$ (X = Cl, Br) in Gegenwart einer Base.

Die Verbindungen der Formeln IV und V sind bis auf die Einzelverbindungen 2-[4-(4-Fluorphenoxy)-phenoxy]-1-methyläthylamin (Verbindung IVa) und 2-[4-(4-Fluorphenoxy)-phenoxy]-1-methylpropylamin (Verbindung IVb) bekannt. Diese neuen Zwischenprodukte können auf dem folgenden Wege durch "reduktive Aminierung" hergestellt werden:

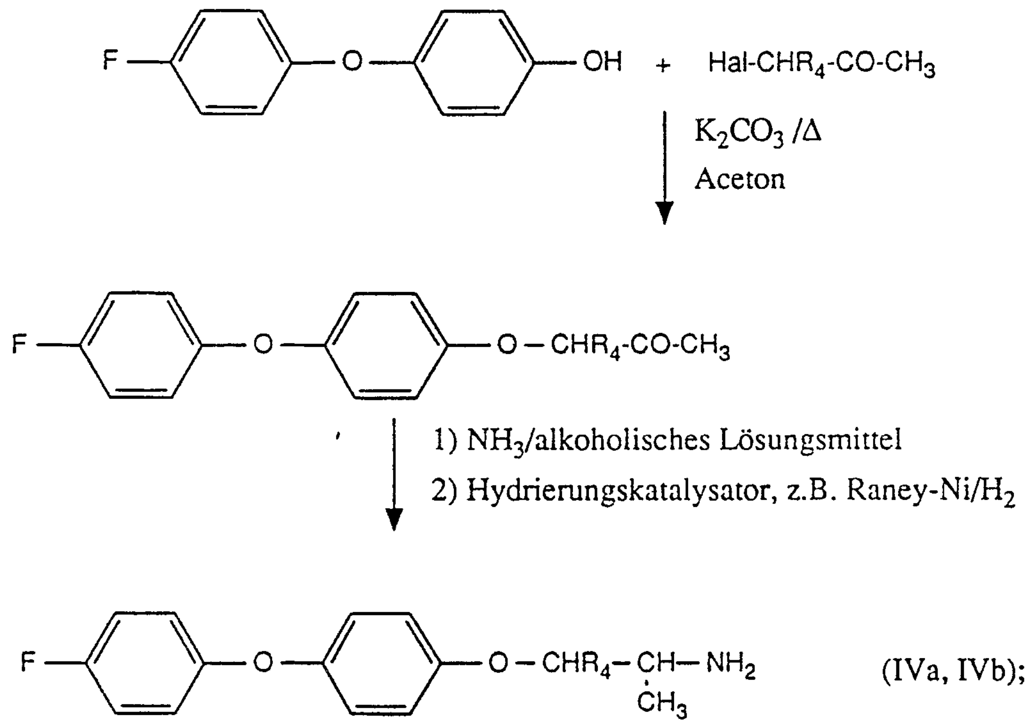


(IVa, IVb);

Hal bedeutet Chlor oder Brom, $R_4$ Wasserstoff oder Methyl. Die Verbindungen IVa und IVb sind neu und bilden zusammen mit dem Verfahren zu ihrer Herstellung ebenfalls einen Gegenstand vorliegender Erfindung.

Die Wirkung der erfindungsgemässen Verbindungen, beziehungsweise der sie enthaltenden Mittel, lässt sich durch Zusatz von anderen Insektiziden und/oder Akariziden wesentlich verbreitern und an gegebene Umstände anpassen. Als solche Zusätze kommen zum Beispiel organische Phosphorverbindungen, Nitrophenole und deren Derivate, Formamidine, Harnstoffe, Carbamate, Pyrethroide, chlorierte Kohlenwasserstoffe und Bacillus thuringiensis-Präparate in Betracht.

Mit besonderem Vorteil kann man die Verbindungen der Formel I auch mit Substanzen kombinieren, welche einen pestizid verstärkenden Effekt ausüben. Beispiele solcher Verbindungen sind unter anderen: Piperonylbutoxid, Propinyläther, Propinyloxime, Propinylcarbamate und Propinylphosphonate, 2-(3,4-Methylendioxyphenoxy)-3,6,9- trioxaundecan oder S,S,S-Tributylphosphorotrithioate.

Die Verbindungen der Formel I werden in unveränderter Form oder vorzugsweise zusammen mit den in der Formulierungstechnik üblichen Hilfsmitteln eingesetzt und werden daher beispielsweise zu Emulsionskonzentraten, direkt versprühbaren oder verdünnbaren Lösungen, verdünnten Emulsionen, Spritzpulvem, löslichen Pulvern, Stäubemitteln, Granulaten oder auch Verkapselungen in zum Beispiel polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierungen, das heisst die den Wirkstoff der Formel I oder Kombinationen dieser Wirkstoffe mit andern Insektiziden oder Akariziden, und gegebenenfalls einen festen oder flüssigen Zusatzstoff enthaltenden Mittel, Zübereitungen oder Zusammensetzungen, werden in bekannter Weise hergestellt, wie durch inniges Vermischen und/oder Vermahlen der Wirkstoffe mit Streckmitteln, wie zum Beispiel mit Lösungmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$-$C_{12}$, wie zum Beispiel Xylolgemische oder substituierte Naphthaline, Phthalsäureester, wie Dibutyl- oder Dioctylphthalat, aliphatische Kohlenwasserstoffe, wie Cyclohexan, Paraffine, Alkohle und Glykole sowie deren Aether und Ester, wie Aethanol, Aethylenglykol, Aethlenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle, wie epoxydiertes Kokosnussöl oder Sojaöl, oder Wasser.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähig Polymerisate zugesetzt werden.

Als gekörnte, adsorptive Granulatträger kommen sowohl poröse Typen, wie zum Beispiel Bimsstein, Ziegelbruch, Sepiolit oder Bentonit, als auch nicht sorptive Trägermaterialien wie Calcit oder Sand in Frage. Darüberhinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach der Art des zu formulierenden Wirkstoffes der Formel I oder der Kombinationen dieser Wirkstoffe und andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen wie wasserlöslich synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie die Natrium- oder Kalium-Salze der Oel- oder Stearinsäure, oder von natürlichen Fettsäuregemischen, die beispielsweise aus Kokosnuss- oder Talgöl gewonnen werden können. Ferner sind auch die Fettsäure-methyl-taurinsalze zu erwähnen.

Häufiger werden jedoch sogenannte Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, zum Beispiel das Natrium- oder Calcium-Salz der Ligninsulfonsäure, des Dodecylschwefelsäureesters oder eines aus natürlichen Fettsäuren hergestellten Fettalkoholsulfatgemisches. Hierher gehären auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8 - 22 C-Atomen. Arylalkylsulfonate sind beispielsweise die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie die Salze des Phosphorsäureesters eines p-Nonylphenol-(4-14)-Aethylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Proplyenglykoleinheit 1 bis 5 Aethylenglykoleinheiten.

Als Beispiele nichtionischer Tenside seien Nonylphenolpolyäthoxyäthanole, Ricinusölpolyglykoläther, Polypropylen-Polyxäthylenoxidaddukte, Tributylphenoxypolyäthoxyäthanol, Polyäthylenglkol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, zum Beispiel das Stearyltrimethylammoniumchlorid oder das Benzyl-di-(2-chloräthyl)-äthylammoniumbromid.

Die in der Formulierungstechnik gebräuchlichen Tenside sind unter anderem in den folgenden Publikationen beschrieben:

"1985 International Mc Cutcheon's Emulsifiers & Detergents", Glen Rock NJ USA, 1985",

H. Stache, "Tensid-Taschenbuch", 2. Aufl., C. Hanser Verlag München, Wien 1981,

M. and J. Ash. "Encyclopedia of Surfactants", Vol. I-III, Chemical Publishing Co., New York, 1980-1981.

Die pestiziden Zübereitungen enthalten in der Regel 0,1 bis 99 % insbesondere 0,1 bis 95 %, des Wirkstoffs der Formel I oder Kombinationen dieses Wirkstoffs mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 10 bis 1000 g Wirkstoff pro Hektar,

vorzugsweise 25 bis 250 g/ha.

Insbesondere setzen sich bevorzugte Formulierungen folgendermassen zusammen: (% = Gewichtsprozent)

Emulgierbare Konzentrate
Aktiver Wirkstoff: 1 bis 50 %, bevorzugt 5 bis 30 %
oberflächenaktives Mittel: 5 bis 30 %, vorzugsweise 10 bis 20 %
flüssiges Trägermittel: 20 bis 94 %, vorzugsweise 50 bis 85 %

Stäube:
Aktiver Wirkstoff: 0,1 bis 10 %, vorzugsweise 0,1 bis 1 %
festes Trägermittel: 99,9 bis 90 %, vorzugsweise 99,9 bis 99 %

Suspensions-Konzentrate
Aktiver Wirkstoff: 5 bis 75 %, vorzugsweise 10 bis 50 %
Wasser: 94 bis 24 %, vorzugsweise 88 bis 30 %
oberflächenaktives Mittel: 1 bis 40 %, vorzugsweise 2 bis 30 %

Benetzbare Pulver
Aktiver Wirkstoff: 0,5 bis 90 %, vorzugsweise 1 bis 80 %
oberflächenaktives Mittel: 0,5 bis 20 %, vorzugsweise 1 bis 15 %
festes Trägermaterial: 5 bis 95 %, vorzugsweise 15 bis 90 %

Granulate
aktiver Wirkstoff: 0,5 bis 30 %, vorzugsweise 3 bis 15 %
festes Trägermittel: 99,5 bis 70 %, vorzugsweise 97 bis 85 %

Die Mittel können auch weitere Zusätze wie Stabilisatoren, Entschäumer, Konservierungmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung. Sie schränken die Erfindung nicht ein.

Beispiel H1: N-Methoxyoxalyl-2-[4-(4-fluorphenoxy)-phenoxy]-äthylcarbaminsäure-äthylester

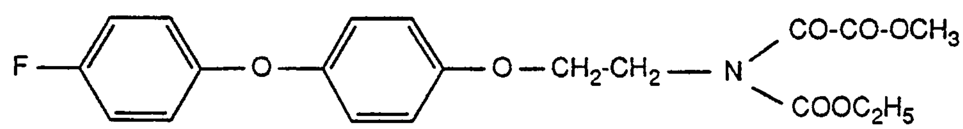

Zu einer Lösung von 10,0 g 2-[4-(4-Fluorphenoxy)-phenoxy]-äthylcarbaminsäure-äthylester und 0,3 g 4-Dimethylaminopyridin in 70 ml 1,2-Dichloräthan tropft man unter Rühren und unter einer Stickstoffatmosphäre bei +80 °C innerhalb von 20 Minuten die Lösung von 7,7 g Oxalsäure-mono-methylester-chlorid in 10 ml 1,2-Dichloräthan. Anschliessend wird das Gemisch für 16 Stunden bei +80 °C gerührt. Nach dem Abkühlen auf Raumtemperatur wird das Reaktionsgemisch nacheinander mit kalter 5%iger Natriumhydrogencarbonat-Lösung, mit 0,1N Salzsäure und schliesslich mit Wasser gewaschen und über Natriumsulfat getrocknet. Das 1,2-Dichloräthan wird im Vakuum vollständig abdestilliert. Der so als Rohprodukt erhaltene N-Methoxyoxalyl-2-[4-(4-fluorphenoxy)-phenoxy]-äthylcarbaminsäure-äthylester kann, falls erwünscht, durch Chromatographie gereinigt werden (Eluierungsmittel: n-Hexan/Diäthyläther, 5:1), $n_D^{20}$: 1,5295.

In gleicher Weise erhält man aus 2-[4-(4-Fluorphenoxy)-phenoxy]-äthyl-carbaminsäureäthylester und Oxalsäure-monoäthylester-chlorid oder Oxalsäure-mono-tert.butylesterchlorid die erfindungsgemässen Verbindungen
N-Aethoxyoxalyl-2-[4-(4-fluorphenoxy)-phenoxy]-äthylcarbaminsäure-äthylester, $n_D^{21}$: 1,5285 , und
N-tert.Butoxyoxalyl-2-[4-(4-fluorphenoxy)-phenoxy]-äthylcarbaminsäure-äthylester, $n_D^{21}$: 1,5200.

Beispiel H2: N-Methoxyoxalyl-2-[4-(4-fluorphenoxy)-phenoxyl-1-methyläthylcarbaminsäure-äthylester

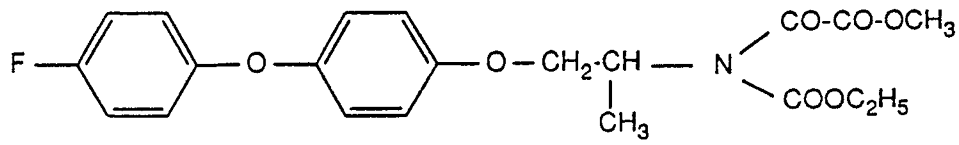

a) 1-[4-(4-Fluorphenoxy)-phenoxy]-2-propanon.

Zu der Lösung von 94,1 g 4-(4-Fluorphenoxy)-phenol in 400 ml Aethylmethylketon setzt man 83 g pulverisiertes Kaliumcarbonat sowie 4 g feinpulveriges Kaliumjodid hinzu und erwarmt auf Rückflusstemperatur. Unter Rühren lässt man innerhalb einer Stunde 64 g frisch destilliertes Choraceton zutropfen und rührt für weitere 2 Stunden bei Rückflusstemperatur. Nach dem Abkühlen wird das Reaktionsgemisch filtriert, das Lösungsmittel im Vakuum abdestilliert und das Rohprodukt aus n-Hexan/Aether (5:1) umkristallisiert, wodurch das reine, farblose 1-[4-(4-Fluorphenoxy)-phenoxy]-2-propanon vom Smp. 56-67° C erhalten wird.

b) 2-Amino-1-[4-(4-fluorphenoxy)-phenoxy]-propan.

26 g 1-[4-(4-Fluorphenoxy)-phenoxy]-2-propanon werden in einem Autoklaven in 250 ml Methanol gelöst und mit 2,4 g Raney-Nickel versetzt. Es werden 17 g flüssiges Ammoniak eingefüllt und dann wird Wasserstoff aufgepresst. Das Reaktionsgemisch wird bei 50 bar und +40° C für 11 Stunden hydriert. Anschliessend werden nochmals 2,4 g Raney-Nickel und 17 g Ammoniak hin- zugefügt und weitere 9 Stunden bei +40° C und 50 bar Wasserstoffdruck bis zum vollständigen Umsatz des Edukts hydriert. Das Reaktionsgemisch wird filtriert. Das Lösungsmittel wird im Vakuum vollständig abdestilliert. Durch Chromatographie an Kieselgel des Rohproduktes (Eluierungsmittel: Diäthyläther/Methanol, 9:1) wird das reine 2-Amino-1-[4-(4-fluorphenoxy)phenoxy]-propan als farblose, ölartige Flüssigkeit mit der Brechung $n_D^{20}$: 1,5531 erhalten.

c) 2-[4-(4-Fluorphenoxy)-phenoxy]-1-methyläthylcarbaminsäure-äthylester.

Zu der Lösung von 13,1 g 2-Amino-1-[4-(4-fluorphenoxy)-phenoxy]-propan, 9,5 g Diisopropyläthylamin und 0,4 g 4-Dimethylaminopyridin in 70 ml Toluol lässt man unter Rühren bei 20-22° C innerhalb von 30 Minuten die Lösung von 5,9 g Chlorameisensäureäthylester in 10 ml Toluol zutropfen. Anschliessend wird für 15 Stunden bei Raumtemperatur gerührt. Zur Aufarbeitung wird das Reaktionsgemisch auf 300 ml Eiswasser gegossen und dreimal mit Aether extrahiert. Die vereinigten organischen Phasen werden zweimal mit 0,2N-Salzsäure und mit Wasser gewaschen. Nach dem Trocknen der organischen Lösung über Natriumsulfat wird das Lösungsmittel abdestilliert und das Rohprodukt durch Chromatographie an Kieselgel gereinigt (Eluierungsmittel: n-Hexan/Diäthyläther 2:1), wodurch der reine 2-[4-(4-Fluorphenoxy)-phenoxy]-1-methyläthylcarbaminsäure-äthylester als farblose Kristalle erhalten wird; Smp. 55-56°.

d) N-Methoxyoxalyl-2-[4-(4-fluorphenoxy)-phenoxy]-1-methyläthylcarbaminsäure-äthylester.

Zu der Lösung von 8,0 g 2-[4-(4-Fluorphenoxy)-phenoxy]-1-methyläthylcarbaminsäureäthylester und 0,3 g 4-Dimethylaminopyridin in 50 ml 1,2-Dichloräthan lässt man unter Rühren und unter einer Stickstoffatmosphäre bei +80° C innerhalb von 20 Minuten die Lösung von 11,8 g Oxalsäure-monomethylester-chlorid in 10 ml 1,2-Dichloräthan eintropfen. Anschliessend wird das Gemisch für 16 Stunden bei +80° C gerührt. Nach dem Abkühlen auf Raumtemperatur wird das Reaktionsgemisch nacheinander mit gekühlter 5%iger Natriumhydrogencarbonat-Lösung, mit 0,1N Salzsäure und schliesslich mit Wasser gewaschen und über Natriumsulfat getrocknet. Das 1,2-Dichlormethan wird im Vakuum vollständig abdestilliert. Der so erhaltene N-Methoxyoxalyl-2-[4-(4-fluorphenoxy)-phenoxy]-1-methyläthylcarbaminsäure-äthyleste r kann, falls erwünscht, an Kieselgel 60 chromatographiert werden (Eluierungsmittel: n-Hexan/Dichlormethan, 2:1); $n_D^{24}$: 1,5230.

<u>Beispiel H3: N-Methoxyoxalyl-2-[4-(4-fluorophenoxy)-phenoxy]-1-methyl-propylcarbaminsäure-äthylester</u>

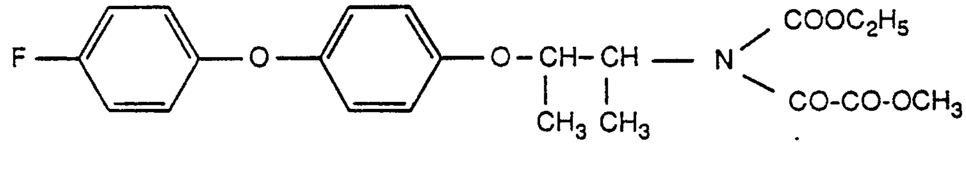

Analog zur im Beispiel H2 beschriebenen Verfahrensweise erhält man

a) aus 4-(4-Fluorphenoxy)-phenol und 2-Chlor-3-butanon als Zwischenprodukt 2-[4-(4-Fluorphenoxy)-phenoxy]-3-butanon hergestellt, $n_D^{20}$: 1,5412;

b) aus 2-[4-(4-Fluorphenoxy)-phenoxy]-3-butanon und Ammoniak in Gegenwart von Raney-Nickel das Zwischenprodukt 2-[4-(4-Fluorphenoxy)-phenoxy]-3-aminobutan, $n_D^{20}$:1,5485;

c) aus 2-[4-(4-Fluorphenoxy)-phenoxy]-3-aminobutan und Chlorameisensäure-äthylester das Zwischenprodukt 2-[4-(4-Fluorphenoxy)-phenoxy]-1-methyl-propylcarbaminsäureäthylester, $n_D^{20}$: 1,5331;

d) 2-[4-(4-Fluorphenoxy)-phenoxy]-1-methyl-propylcarbaminsäure-äthylester und Oxalsäure-monomethylesterchlorid den erfindungsgemässen N-Methoxyoxalyl-2-[4-(4-fluorphenoxy)-phenoxy]-1-methyl-propylcarbaminsäure-äthylester, $n_D^{21}$: 1,5201.

Beispiel H4: N-Methoxyoxalyl-2-[4-(4-fluorphenoxy)-phenoxy]-äthylcarbaminsäure-methylester

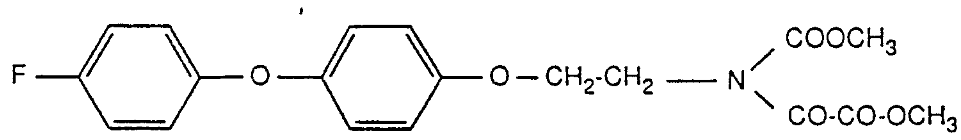

a) Zu einer Lösung von 2,6 g 4-(4-Fluorphenoxy)-phenol in 120 ml Dimethylformamid gibt man 3,6 g Kaliumcarbonatpulver, 1,5 g fein gepulvertes Kaliumjodid und 27 g 2-Chloroäthylcarbaminsäure-methylester zu und erhitzt das Reaktionsgemisch für 15 Stunden auf 95 °C. Hierauf wird das abgekühlte Reaktionsgemisch auf Eiswasser gegossen und wiederholt mit Aether extrahiert. Die vereinigten Aetherphasen werden mit Wasser gewaschen, über Natriumsulfat getrocknet und das Lösungsmittel wird vollständig abdestilliert. Das Rohprodukt wird nach Filtration über Kiesel aus Diäthyläther/Hexan umkristallisiert, wodurch der 2-[4-(4-Fluorphenoxy)-phenoxy]-äthylcarbaminsäuremethylester vom Smp. 64-66 °C erhalten wird.

Analog werden aus den 2-Chloräthylcarbaminsäure-n-propyl-, -isopropyl- und -n-butyl-estern und 4-(4-Fluorphenoxy)-phenol die folgenden Carbaminsäure-alkylester hergestellt:
2-[4-(4-Fluorphenoxy)-phenoxy]-äthylcarbaminsäure-n-propylester, Smp. 73-74 °C,
2-[4-(4-Fluorphenoxy)-phenoxy]-äthylcarbaminsäure-isopropylester, Smp. 72-74 °C,
2-[4-(4-Fluorphenoxy)-phenoxy]-äthylcarbaminsäure-n-butylester, Smp. 63-64 °C,

b) Analog zu Arbeitsweise des Beispiels H1 werden aus den unter a) hergestellten Carbaminsäure-alkylestern durch Umsetzen mit Oxalsäure-monomethylester-chlorid die folgenden erfindungsgemässen N-Alkoxyoxalyl-2-[4-(4-fluorphenoxy)-phenoxy]-äthylcarbaminsäure-alkylester hergestellt:
N- Methoxyoxalyl-2-[4-(4-fluorphenoxy)-phenoxy]-äthylcarbaminsäure-methylester, $n_D^{20}$: 1,5377;
N- Aethoxyoxalyl-2-[4-(4-fluorphenoxy)-phenoxy]-äthylcarbaminsäure-n-propylester, $n_D^{20}$: 1,5249;
N-Aethoxyoxalyl-2-[4-(4-fluorphenoxy)-phenoxy]-äthylcarbaminsäure-isopropylester, $n_D^{20}$: 1,5226 und
N- Methoxyoxalyl-2-[4-(4-fluorphenoxy)-phenoxy]-äthylcarbaminsäure-n-butylester, $n_D^{20}$: 1,5267.

Beispiel H5: N-Aethoxycarbonyl-2-[4-(4-fluorphenoxy)-phenoxy]-äthylcarbaminsäure-äthylester

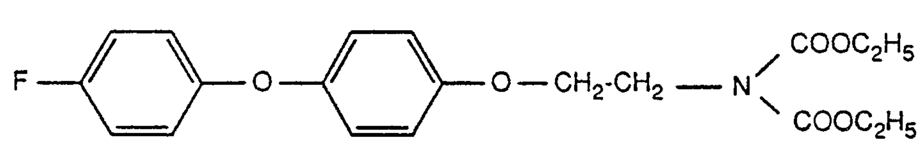

1,56 g einer 55 %igen Natriumhydrid-Dispersion in Mineralöl werden wiederholt mit n-Hexan gewaschen und in 30 ml Tetrahydrofuran suspendiert. Zu dieser Suspension lässt man bei Raumtemperatur unter Rühren die Lösung von 11,4 g 2-[4-(4-Fluorphenoxy)phenoxy]-äthylcarbaminsäure-äthylester in 30 ml Tetrahydrofuran zutropfen und rührt weitere 4 Stunden bei Raumtemperatur bis das Natriumhydrid vollständig umgesetzt ist. Hierauf lässt man bei 0-5 °C innerhalb von 10 Minuten die Lösung von 4,7 g frisch destilliertem Chlorameisensäureäthylester in 10 ml Tetrahydrofuran zutropfen und rührt weitere 50 Minuten bei Raumtemperatur. Nun wird das Reaktionsgemisch auf Eiswasser gegossen und wiederholt mit Aether extrahiert. Die vereinigten Aetherphasen wäscht man zweimal mit kalter, gesättigter Natriumhydrogencarbonatlösung, anschliessend mit Wasser und Natriumchloridlösung, trocknet die organische Phase über

Natriumsulfat und destilliert das Lösungsmittel ab. Das Rohprodukt wird an Kieselgel chromatographisch weiter gereinigt (Eluierungsmittel: n-Hexan/Diäthyläther, 5:1) wodurch der N-Aethoxycarbonyl-2-[4-(4-fluorphenoxy)-phenoxy]-äthylcarbaminsäure-äthylester als farbloses, viskoses Oel erhalten wird; $n_D^{23}$: 1,5257.

Analog werden hergestellt:

Aus 2-[4-(4-Fluorphenoxy)-phenoxy]-äthylcarbaminsäure-äthylester und Acetylchlorid der N-Acetyl-2-[4-(4-fluorphenoxy)-phenoxy]-äthylcarbaminsäure-äthylester, Smp. 52-53° C; und

aus 2-[4-(4-Fluorphenoxy)-phenoxy]-äthylcarbaminsäure-äthylester und frisch destilliertem Diäthylcarbamoylchlorid der N-Diäthylcarbamoyl-2-[4-(4-fluorphenoxy)phenoxy]-äthylcarbaminsäure-äthylester, $n_D^{20}$: 1,5318.

Beispiel H6: N-(N-Morpholino-oxalyl)-2-[4-(4-fluorphenoxy)-phenoxy]-äthylcarbaminsäure-äthylester

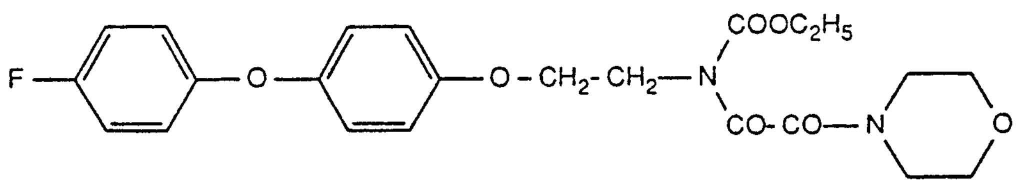

a) Zu der Lösung von 30,5 g 2-[4-(4-Fluorphenoxy)-phenoxy]-äthylcarbaminsäureäthylester in 100 ml 1,2-Dichloräthan lässt man bei Raumtemperatur unter Rühren 25,4 g Oxalylchlorid zugtropfen und erhitzt für 3 Stunden auf Rückflusstemperatur bis kein Chlorwasserstoffgas mehr entwickelt wird. Nun wird unter einer Stickstoffatmosphäre das Lösungsmittel und das überschüssige Oxalylchlorid adestilliert und der so erhaltene N-Chloroxalyl-2-[4-(4-fluorphenoxy)-phenoxy]-äthylcarbaminsäure-äthylester aus n-Pentan umkristallisiert. Man erhält farblose Kristalle vom Smp. 48-50° C.

b) Zu der Lösung von 8,2 g N-Chloroxalyl-2-[4-(4-fluorphenoxy)-phenoxy]-äthylcarbaminsäure-äthylester in 80 ml Toluol lässt man unter Rühren bei 0-5° C innerhalb von 30 Minuten die Lösung von 5,2 g Morpholin in 20 ml Toluol zutropfen. Anschliessend wird noch für 2 Stunden bei Raumtemperatur gerührt. Nun wird das Reaktionsgemisch nacheinander wiederholt mit eiskalter 1N-Salzsäure, mit 10 %iger Natriumhydrogencarbonatlösung und mit Wasser gewaschen, über Natriumsulfat getrocknet und das Lösungsmittel vollständig abdestilliert. Schliesslich wird der so erhaltene farblose, viskose N-(N-Morpholino-oxalyl)-2-[4-(4-fluorphenoxy)-phenoxy]-äthylcarbaminsäure-äthylester in Hochvakuum vollständig entgast, $n_D^{21}$: 1,5471.

Analog werden aus N-Chloroxalyl-2-[4-(4-fluorphenoxy)-phenoxy]-äthylcarbaminsäureäthylester und Piperidin oder Diäthylamin die folgenden Verbindungen hergestellt:

N-(N-Piperidino-oxalyl)-2-[4-(4-fluorphenoxy)-phenoxy]-äthylcarbaminsäure-äthylester, $n_D^{21}$:1,5451; und

N-(N-Diäthylamino-oxalyl)-2-[4-(4-fluorphenoxy)-phenoxy]-äthylcarbaminsäureäthylester, $n_D^{20}$:1,5349.

In analoger Weise kann man die folgenden Wirkstoffe der Formel I erhalten:

Tabelle 1:

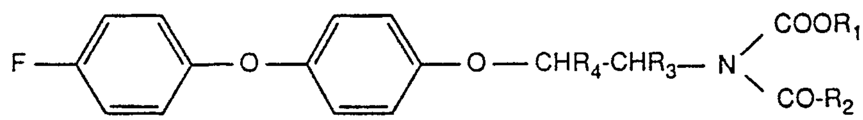

| Verb. Nr. | $R_1$ | $R_2$ | $R_3$ | $R_4$ | Physikalische Daten |
|---|---|---|---|---|---|
| 1.01 | $C_2H_5$ | $COOC_2H_5$ | H | H | $n_D^{21}$ : 1,5285 |
| 1.02 | $C_2H_5$ | $COOC_4H_9$-t | H | H | $n_D^{21}$ : 1,5200 |
| 1.03 | $C_2H_5$ | $COOCH_3$ | H | H | $n_D^{20}$ : 1,5295 |
| 1.04 | $C_2H_5$ | $COOC_2H_5$ | $CH_3$ | H | |
| 1.05 | $CH_3$ | $COOCH_3$ | H | H | $n_D^{20}$ : 1,5377 |
| 1.06 | $C_3H_7$-n | $COOC_2H_5$ | H | H | $n_D^{20}$ : 1,5249 |
| 1.07 | $C_4H_9$-n | $COOCH_3$ | H | H | $n_D^{20}$ : 1,5267 |
| 1.08 | $C_2H_5$ | $COOCH_3$ | $CH_3$ | H | $n_D^{24}$ : 1,5230 |
| 1.09 | $C_2H_5$ | $COOCH_3$ | $CH_3$ | $CH_3$ | $n_D^{21}$ : 1,5201 |
| 1.10 | $C_2H_5$ | $CH_3$ | H | H | Smp. 52-53°C |
| 1.11 | $C_2H_5$ | $C_3H_7$-n | H | H | |
| 1.12 | $C_2H_5$ | $OC_2H_5$ | H | H | $n_D^{23}$ : 1,5257 |
| 1.13 | $C_2H_5$ | $OCH_3$ | H | H | |
| 1.14 | $C_2H_5$ | $OC_4H_9$-n | H | H | |
| 1.15 | $C_2H_5$ | $COOC_8H_{17}$-n | H | H | |
| 1.16 | $C_2H_5$ | $COOC_3H_7$-i | H | H | |
| 1.17 | $C_2H_5$ | $-N(CH_3)_2$ | H | H | |
| 1.18 | $C_2H_5$ | $-N(C_2H_5)_2$ | H | H | $n_D^{20}$: 1,5318 |
| 1.19 | $C_2H_5$ | $CO-N(C_2H_5)_2$ | H | H | $n_D^{20}$: 1,5349 |
| 1.20 | $C_2H_5$ | 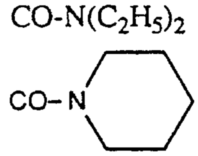 | H | H | $n_D^{21}$ : 1,5451 |

Tabelle 1: (Fortsetzung)

| Verb. Nr. | $R_1$ | $R_2$ | $R_3$ | $R_4$ | Physikalische Daten |
|---|---|---|---|---|---|
| 1.21 | $C_2H_5$ | $CO\text{-}N(C_4H_9\text{-}n)_2$ | H | H | |
| 1.22 | $C_2H_5$ | $CO\text{-}NHC_4H_9\text{-}n$ | H | H | |
| 1.23 | $C_2H_5$ | $CO\text{-}NH\text{-}CH_2\text{-}C_6H_5$ | H | H | |
| 1.24 | $C_2H_5$ | 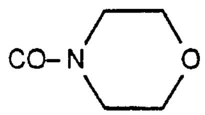 | H | H | $n^{21}_{D}$ : 1,5471 |
| 1.25 | $C_2H_5$ | $CO\text{-}N(CH_3)\text{-}C_6H_5$ | H | H | |
| 1.26 | $C_2H_5$ | $CO\text{-}NH\text{-}C_6H_5$ | H | H | |
| 1.27 | $C_3H_7\text{-}i$ | $COOC_2H_5$ | H | H | $n^{20}_{D}$ : 1,5226 |

Formulierungsbeispiele für flüssige Wirkstoffe der Formel I (% = Gewichtsprozent)

| F1. Emulsions-Konzentrate | a) | b) | c) |
|---|---|---|---|
| Wirkstoff Nr. 1.08 oder 1.02 | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| F2. Lösungen | a) | b) | c) | d) |
|---|---|---|---|---|
| Wirkstoff Nr. 1.02 | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190° C) | - | - | 94 % | - |

Die Lösungen sind zur Anwendung in Form kleinster Tropfen geeignet.

| F3. Granulate | a) | b) |
|---|---|---|
| Wirkstoff Nr. 1.03 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| F4. Stäubemittel | | |
|---|---|---|
| Wirkstoff Nr. 1.01 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| F5. Sprizpulver | | | |
|---|---|---|---|
| Wirkstoff Nr. 1.10 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F6. Emulsions-Konzentrat | |
|---|---|
| Wirkstoff Nr. 1.10 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 50 % |

Aus diesem Konzentrat können durch Verdünnen mit Wasser Emulsionen jeder gewünschten Konzentration hergestellt werden.

| F7. Stäubemittel | a) | b) |
|---|---|---|
| Wirkstoff Nr. 1.10 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| F8. Extruder-Granulat | |
|---|---|
| Wirkstoff Nr. 1.10 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

| F9. Umhüllungs-Granulat | |
|---|---|
| Wirkstoff Nr. 1.10 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| F10. Suspensions-Konzentrat | |
|---|---|
| Wirkstoff Nr. 1.10 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

In den nachfolgenden biologischen Beispielen bedeutet eine gute Wirkung, dass der erwünschte Effekt zu mindestens 50 bis 60 % eintritt.

Beispiel B1: Wirkung gegen Boophilus microplus

Vollgesogene adulte Zecken Weibchen werden auf eine PVC Platte aufgeklebt und mit einem Wattebausch überdeckt. Zur Behandlung werden die Testtiere mit 10 ml einer wässrigen Testlösung die 125 ppm des zu prüfenden Wirkstoffes enthält übergossen. Anschliessend wird der Wattebausch entfernt und die Zecken werden für 4 Wochen zur Eiablage inkubiert. Die Wirkung gegen Boophilus microplus zeigt sich

eintweder beim Weibchen als Mortalität oder Sterilität, oder bei den Eiern als ovizide Wirkung.

Verbindungen gemäss Tabelle 1 zeigen in diesem Test gute Wirkung gegen Boophilus microplus. Insbesondere die Verbindungen 1.01, 1.02, 1.03, 1.08, 1.10 und 1.12 zeigen eine Wirkung über 80 %.

Beispiel B2: Ovizide Wirkung auf Cydia pomonella

Auf Filterpapier abgelegte Eier von Cydia pomonella werden für eine kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu den unbehandelten Kontrollansätzen ausgewertet (% Schlupfreduktion).

Verbindungen gemäss Tabelle 1 zeigen in diesem Test gute Wirkung gegen Cydia pomonella.

Beispiel B3: Wirkung geben Dermanyssus gallinae

In einem oben offenen Glasbehälter werden 2 bis 3 ml einer 10 ppm Wirkstoff enthaltenden Lösung und ca. 200 Milben in unterschiedlichen Entwicklungsstadien gegeben. Anschliessend wird der Behälter mit einem Wattebausch verschlossen, 10 Minuten lang bis zur vollständigen Benetzung der Milben geschüttelt und dann kurzfristig umgekehrt, damit die restliche Testlösung von der Watte aufgenommen werden kann. Nach 3 Tagen wird die Mortalität der Milben durch Auszählen der toten Individuen ermittelt und in Prozent angegeben.

Verbindungen gemäss Tabelle 1 zeigen eine gute Wirkung gegen Dermanyssus gallinae.

Beispiel B4: Ovizide Wirkung auf Adoxophyes reticulana

Auf Filterpapier abgelegte Eier von Adoxophyes reticulana werden für eine kurze Zeit in eine acetonisch-wässrige Testlösung, die 400 ppm des zu prüfenden Wirkstoffes enthält, eingetaucht. Nach dem Antrocknen der Test-Lösung werden die Eier in Petrischalen inkubiert. Nach 6 Tagen wird der prozentuale Schlupf der Eier im Vergleich zu unbehandelten Kontrollansätzen ausgewertet (% Schlupfreduktion).

Die Verbindungen gemäss Tabelle 1 zeigen in diesem Test gute Wirkung gegen Adoxophyes reticulana. Insbesondere die Verbindungen 1.01 und 1.02 zeigen auch bei 12,5 ppm noch eine Wirkung über 90 %.

Beispiel B5: Ovizide Wirkung auf Lobesia botrana

Auf Filterpapier abgelegte Eier von Lobesia botrana werden für eine kurze Zeit in eine acetonisch-wässrige Testlösung, die 400 ppm des zu prüfenden Wirkstoffes enthält, eingetaucht. Nach dem Antrocknen der Test-Lösung werden die Eier in Petrischalen inkubiert. Nach 6 Tagen wird der prozentuale Schlupf der Eier im Vergleich zu unbehandelten Kontrollansätzen ausgewertet (% Schlupfreduktion).

Die Verbindungen gemäss Tabelle 1 zeigen in diesem Test gute Wirkung gegen Lobesia botrana. Insbesondere die Verbindungen 1.01 und 1.02 zeigen auch bei 12,5 ppm noch eine Wirkung über 90 %.

Beispiel B6: Wirkung gegen Aonidiella aurantii

Kleine Kartoffelknollen werden mit Wanderlarven ("Crawlern") von Aonidiella aurantii (rote Citrus-Schildlaus) besiedelt. Nach etwa 2 Wochen werden die Kartoffeln in eine wässrige Emulsions- resp. Suspensions-Spritzbrühe getaucht, die den zu prüfenden Wirkstoff in einer Konzentration von 400 ppm enthält. Nach dem Abtrocknen der so behandelten Kartoffelknollen werden diese in Plastikbehälter inkubiert. Zur Auswertung wird 10-12 Wochen später die Ueberlebensrate der Wanderlarven der ersten Folgegeneration der behandelten Schildlaus-Population mit derjenigen der unbehandelten Kontrollansätze verglichen.

Verbindungen gemäss Tabelle 1 zeigen in diesem Test gute Wirkung gegen Aonidiella aurantii.

Beispiel B7: Chemosterilisierungs-Wirkung auf Nilaparvata lugens

Der Test wird an wachsenden Pflanzen durchgeführt. Jeweils 4 Reispflanzen (Dicke des Stengels 8 mm, Höhe ca. 20 cm) werden in Töpfe (Durchmesser von 8 cm) eingepflanzt. Die Pflanzen werden mit einer wässrigen Emulsionszubereitung enthaltend 400 ppm des Wirkstoffes tropfnass besprüht. Nach dem Antrocknen des Spritzbelages wird jede Pflanze mit frisch geschlüpften adulten Weibchen und Männchen von Nilaparvata besiedelt. Um die Tiere am Entweichen zu hindern, wird über jede besiedelte Pflanze ein Glaszylinder gestülpt und dieser mit einem Gaze-Deckel abgedeckt. Die adulten Tiere bleiben 5 Tage zur Eiablage auf der behandelten Pflanze und werden danach entfernt. Die Reispflanzen mit den abgelegten Eiern werden dann während 14 Tagen bei + 28° C, 70 % relativer Feuchtigkeit und einer 14-stündigen Photoperiode (10'000 Lux) inkubiert. Die in diesem Zeitraum geschlüpften jungen Nymphen ($F_1$-Generation) werden ausgezählt. Aus dem Vergleich der Anzahl geschlüpfter Nymphen auf den behandelten Pflanzen zu den auf unbehandelten Kontrollpflanzen geschlüpften Tieren wird die prozentuale Reduktion der Nachkommenschaft (Chemosterilisations-Effekt = % Wirkung) bestimmt.

Die Verbindungen der Tabelle 1 zeigen eine gute Wirkung in obigem Test. Die Verbindungen 1.01 und 1.02 sind bei Konzentrationen von 6 ppm zu 100 % wirksam; bei 1,5 ppm noch zu 95 %.

Beispiel B8: Populationshemmende Wirkung auf Nephotettix cincticeps

Der Test wird an wachsenden Reispflanzen durchgeführt. Jeweils 20 Reispflanzen (Dicke des Stengels 1 mm, Höhe ca. 20 cm) werden in Porzellantöpfe von 8 cm Durchmesser eingepflanzt. Die Pflanzen werden mit einer wässrigen Emulsionszubereitung, enthaltend 400 ppm des Wirkstoffes tropfnass besprüht. Nach dem Antrocknen des Spritzbelages wird jeder Topf mit adulten Weibchen und adulten Männchen (frisch geschlüpft) besiedelt. Um die Tiere am Entweichen zu hindern, wird über jeden besiedelten Topf ein Plexiglaszylinder gestülpt und dieser mit einem Gazedeckel abgedeckt. Die adulten Tiere bleiben 5 Tage zur Eiablage auf den behandelten Pflanzen und werden danach entfernt. Die Reispflanzen mit den abgelegten Eiern werden während 14 Tagen bei + 28° C, 70 % relativer Feuchtigkeit und einer 14-stündigen Photoperiode (10'000 Lux) inkubiert. Die in diesem Zeitraum geschlüpften jungen Nymphen ($F_1$ Generation) werden ausgezählt. Aus dem Vergleich der Anzahl geschlüpfter Nymphen auf den behandelten Pflanzen zu den auf unbehandelten Kontrollpflanzen geschlüpften Tieren wird die prozentuale Reduktion der Nachkommenschaft (% Populationshemmung) bestimmt.

Die Verbindungen der Tabelle 1 zeigen eine gute Wirkung in obigem Test.

Beispiel B9: Wirkung gegen Bemisia tabaci

Buschbohnen-Pflanzen werden in Gazekäfige gestellt und mit Adulten von Bemisia tabaci (Weisse Fliege) besiedelt. Nach erfolgter Eiablage werden alle Adulten entfernt und 10 Tage später die Pflanzen mit den darauf befindlichen Nymphen mit einer wässrigen Emulsions-Spritzbrühe der zu prüfenden Wirkstoffe (Konzentration 400 ppm) behandelt. Die Auswertung erfolgt 14 Tage nach der Wirkstoff-Applikation auf %-Schlupf im Vergleich zu den unbehandelten Kontrollansätzen.

Die Verbindungen der Tabelle 1 zeigen gute Wirkung in diesem Test. Die Verbindungen 1.01 und 1.02 sind bei Konzentrationen von 10 ppm zu 100 % wirksam.

## Ansprüche

1. N-Acyl-2-[4-(4-fluorphenoxy)-phenoxy]-äthylcarbaminsäureester der Formel

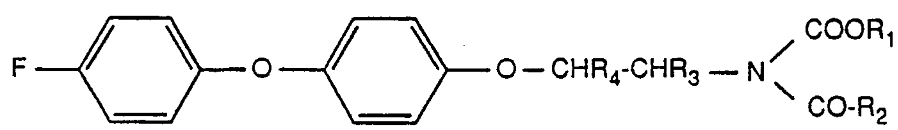 (I),

worin

$R_1$ für $C_1$-$C_8$-Alkyl, $C_3$-$C_4$-Alkenyl oder $C_3$-$C_4$-Alkinyl,
$R_2$ für $C_1$-$C_8$-Alkyl, $C_1$-$C_8$-Alkoxy, -CO-$R_5$ oder -$NR_6R_7$,
$R_3$ und $R_4$ unabhängig voneinander für Wasserstoff oder Methyl,

$R_5$ für $C_1$-$C_8$-Alkoxy oder -$NR_8R_9$,
$R_6$ für $C_1$-$C_4$-Alkyl,
$R_7$ für $C_1$-$C_4$-Alkyl oder
$R_6$ und $R_7$ zusammen für eine $C_4$-$C_6$-Alkylenkette, welche durch Sauerstoff, Schwefel oder -$NCH_3$- unterbrochen sein kann,
$R_8$ für Wasserstoff oder $C_1$-$C_4$-Alkyl und
$R_9$ für Wasserstoff, $C_1$-$C_4$-Alkyl, Benzyl, Phenyl oder durch Halogen oder Methyl substituiertes Phenyl oder $R_8$ und $R_9$ zusammen für eine $C_4$-$C_6$-Alkylenkette, welche durch Sauerstoff, Schwefel oder -$NCH_3$- unterbrochen sein kann, stehen.

2. Verbindungen gemäss Anspruch 1, dadurch gekennzeichnet, dass $R_1$ für $C_1$-$C_4$-Alkyl, $R_2$ für $C_1$-$C_4$-Alkyl, $C_1$-$C_4$-Alkoxy, $C_1$-$C_4$-Alkoxycarbonyl, Di-($C_1$-$C_4$-alkyl)-amino oder -$CONR_8R_9$ stehen, wobei $R_8$ $C_1$-$C_4$-Alkyl, $R_9$ Wasserstoff, $C_1$-$C_4$-Alkyl oder $R_8$ und $R_9$ zusammen eine $C_1$-$C_6$-Alkylenkette welche durch Sauerstoff unterbrochen sein kann, bedeuten.

3. Verbindungen gemäss Anspruch 2, dadurch gekennzeichnet, dass $R_2$ für $C_1$-$C_4$-Alkyl, $C_1$-$C_4$-Alkoxy, $C_1$-$C_4$-Alkoxycarbonyl, Di-($C_1$-$C_4$-alkyl)-aminocarbonyl oder $C_4$-$C_6$-Alkylenaminocarbonyl steht.

4. Verbindungen gemäss Anspruch 1, dadurch gekennzeichnet, dass $R_1$ für $C_1$-$C_4$-Alkyl, $R_2$ für $C_1$-$C_4$-Alkyl, $C_1$-$C_4$-Alkoxy, $C_1$-$C_4$-Alkoxycarbonyl oder Di-($C_1$-$C_2$-alkyl)-aminocarbonyl, $R_3$ für Methyl und $R_4$ für Wasserstoff stehen.

5. Verbindungen gemäss Anspruch 1 ausgewählt aus der Gruppe

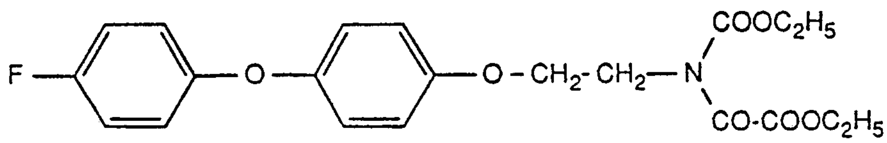

,

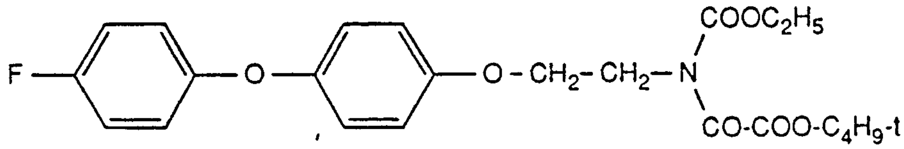

,

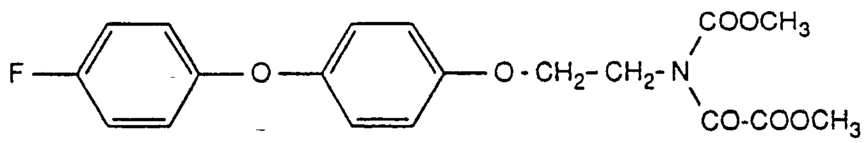

,

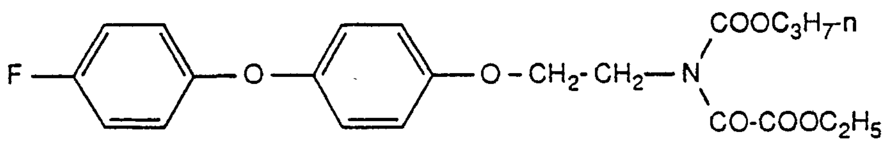

,

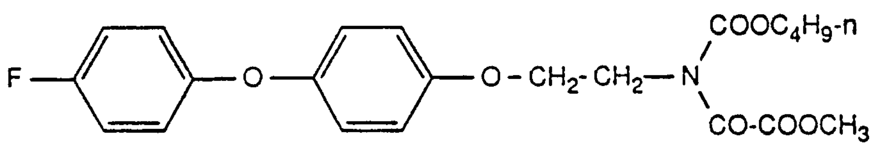

,

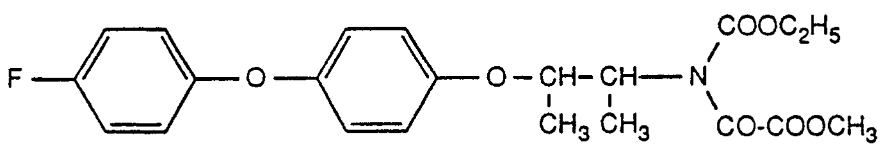

,

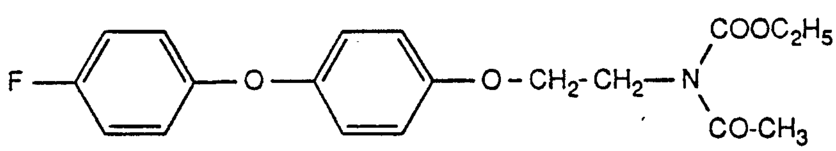

,

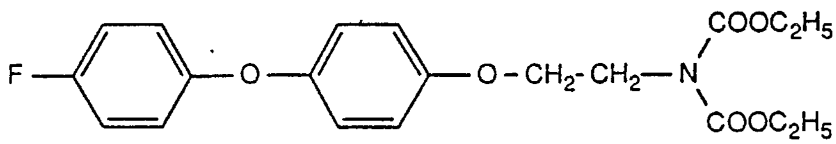

,

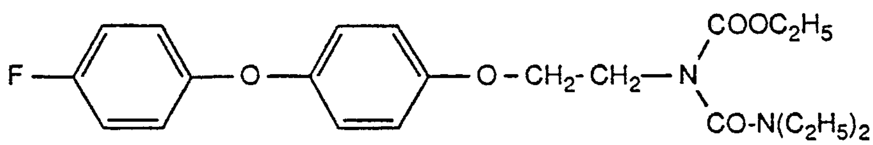

,

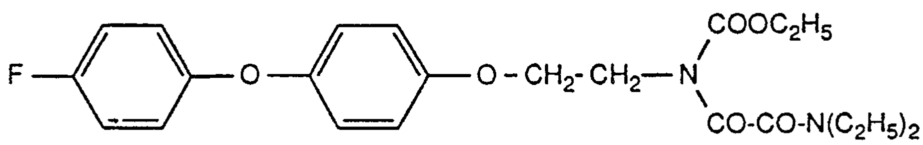

,

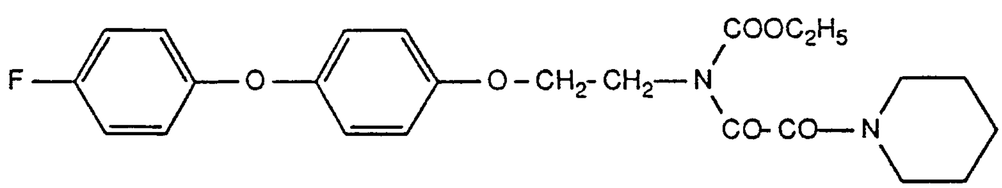

,

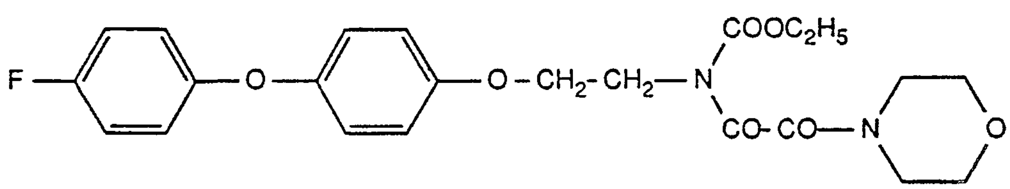

,

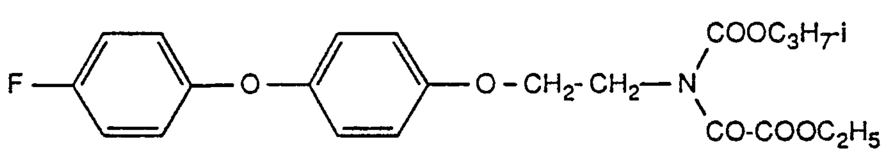

,

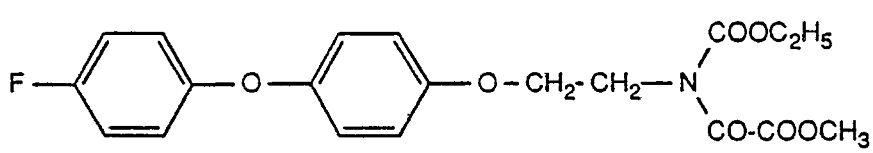

und

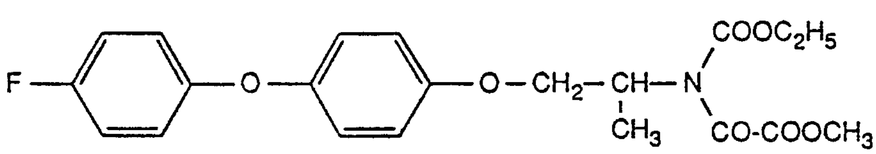

.

6. Verfahren zur Herstellung der Verbindungen der Formel I, dadurch gekennzeichnet, dass man einen Fluorphenoxyphenoxyäthylcarbaminsäureester der Formel II

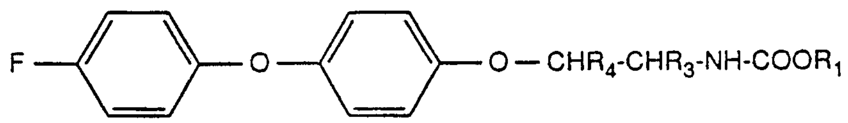

(II)

worin $R_1$, $R_3$ und $R_4$ die unter Formel I angegebenen Bedeutungen haben, mit einer Verbindung der Formel III

$X\text{-}CO\text{-}R_2$ (III)

worin $R_2$ die unter Formel I angegebene Bedeutung hat, und X für Chlor, Brom, $-O\text{-}CO\text{-}R_2$ oder $-O\text{-}CO(C_1\text{-}C_4\text{-Alkyl})$ steht, acyliert.

7. Verfahren gemäss Anspruch 6, dadurch gekennzeichnet, dass man die Acylierung in Gegenwart einer Base durchführt.

8. Schädlingsbekämpfungsmittel, welches als aktive Komponente eine Verbindung der Formel I enthält

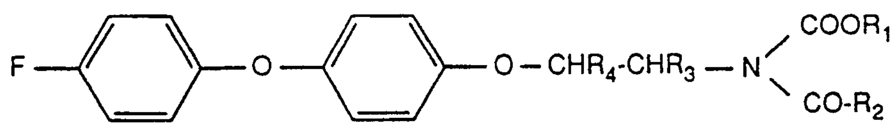

(I),

worin
$R_1$ für $C_1$-$C_8$-Alkyl, $C_3$-$C_4$-Alkenyl oder $C_3$-$C_4$-Alkinyl,
$R_2$ für $C_1$-$C_8$-Alkyl, $C_1$-$C_8$-Alkoxy, $-CO-R_5$ oder $-NR_6R_7$,
$R_3$ und $R_4$ unabhängig voneinander für Wasserstoff oder Methyl,
$R_5$ für $C_1$-$C_8$-Alkoxy oder $-NR_8R_9$,
$R_6$ für $C_1$-$C_4$-Alkyl,
$R_7$ für $C_1$-$C_4$-Alkyl oder
$R_6$ und $R_7$ zusammen für eine $C_4$-$C_6$-Alkylenkette, welche durch Sauerstoff, Schwefel oder $-NCH_3$- unterbrochen sein kann,
$R_8$ für Wasserstoff oder $C_1$-$C_4$-Alkyl und
$R_9$ für Wasserstoff, $C_1$-$C_4$-Alkyl, Benzyl, Phenyl oder durch Halogen oder Methyl substituiertes Phenyl oder
$R_8$ und $R_9$ zusammen für eine $C_4$-$C_6$-Alkylenkette, welche durch Sauerstoff, Schwefel oder $-NCH_3$- unterbrochen sein kann, stehen.

9. Verwendung einer Verbindung der Formel I

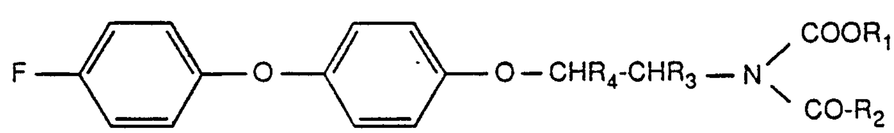

(I),

worin
$R_1$ für $C_1$-$C_8$-Alkyl, $C_3$-$C_4$-Alkenyl oder $C_3$-$C_4$-Alkinyl,
$R_2$ für $C_1$-$C_8$-Alkyl, $C_1$-$C_8$-Alkoxy, $-CO-R_5$ oder $-NR_6R_7$,
$R_3$ und $R_4$ unabhängig voneinander für Wasserstoff oder Methyl,
$R_5$ für $C_1$-$C_8$-Alkoxy oder $-NR_8R_9$,
$R_6$ für $C_1$-$C_4$-Alkyl,
$R_7$ für $C_1$-$C_4$-Alkyl oder
$R_6$ und $R_7$ zusammen für eine $C_4$-$C_6$-Alkylenkette, welche durch Sauerstoff, Schwefel oder $-NCH_3$- unterbrochen sein kann,
$R_8$ für Wasserstoff oder $C_1$-$C_4$-Alkyl und
$R_9$ für Wasserstoff, $C_1$-$C_4$-Alkyl, Benzyl, Phenyl oder durch Halogen oder Methyl substituiertes Phenyl oder
$R_8$ und $R_9$ zusammen für eine $C_4$-$C_6$-Alkylenkette, welche durch Sauerstoff, Schwefel oder $-NCH_3$-

unterbrochen sein kann, stehen, zur Bekämpfung von Schädlingen.

10. Verwendung gemäss Anspruch 9 zur Bekämpfung von Arthropoden, insbesondere von Insekten und Arachniden.

11. Verwendung gemäss Anspruch 10 zur Bekämpfung von phytophagen Schadinsekten und -milben einschliesslich ihrer Larven und Eier.

12. Verwendung gemäss Anspruch 10 zur Chemosterilisierung pflanzenschädigender Schadinsekten und -milben.

13. Die Zwischenprodukte der Formel IVa

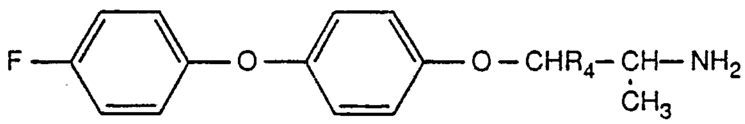

(IVa)

oder der Formel IVb

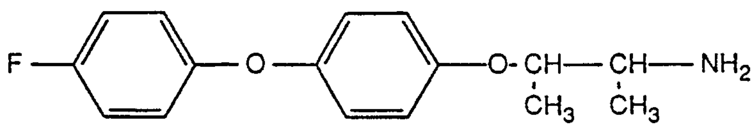

(IVb).

14. Verfahren zur Herstellung der Verbindungen IVa und IVb gemäss Anspruch 13, dadurch gekennzeichnet, dass man 4-(4-Fluorphenoxy)-phenol mit einer Verbindung der Formel Hal-$CHR_4$-CO-$CH_3$, worin $R_4$ Wasserstoff oder Methyl und Hal Cl oder Br bedeuten, in Gegenwart eines Alkalicarbonats erhitzt, das erhaltene 4-(4-Fluorphenoxy)-phenoxy-aceton der Formel

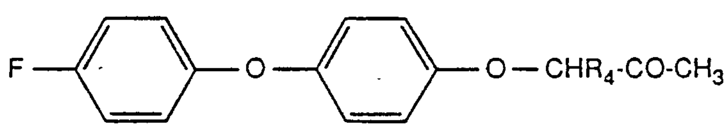

worin $R_4$ Wasserstoff oder Methyl bedeutet, in Gegenwart von einem Hydrierungskatalysator, mit Ammoniak und Wasserstoff unter Druck behandelt.

15. Verfahren zur Herstellung der Verbindungen der Formel I, worin $R_2$ für die Gruppe -CO-$R_5$ steht, dadurch gekennzeichnet, dass man die Verbindung der Formel II nach Anspruch 6 zunächst in einem inerten Lösungsmittel mit Oxalylchlorid umsetzt, und das entstehende Zwischenprodukt der Formel

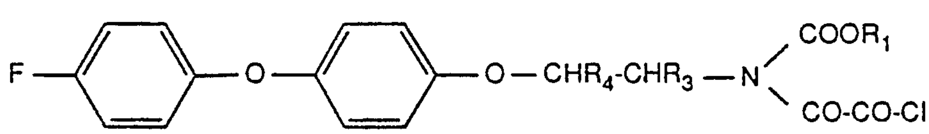

worin $R_1$, $R_3$ und $R_4$ die unter Formel I gegebenen Bedeutungen haben, in Gegenwart einer Base mit einem Alkohol oder einem Amin der Formel

H-$R_5$

worin $R_5$ die unter Formel I gegebene Bedeutung hat, umsetzt.

Patentansprüche für folgenden Vertragsstaat : ES

1. Schädlingsbekämpfungsmittel, welches als aktive Komponente einen N-Acyl-2-[4-(4-fluorphenoxy)-phenoxy]-äthylcarbaminsäureester der Formel

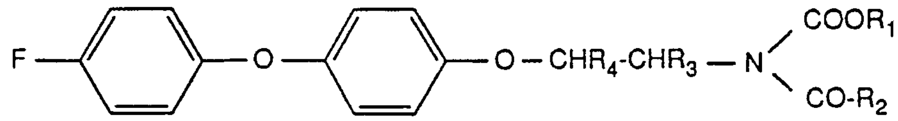 (I),

enthält, worin

$R_1$ für $C_1$-$C_8$-Alkyl, $C_3$-$C_4$-Alkenyl oder $C_3$-$C_4$-Alkinyl,
$R_2$ für $C_1$-$C_8$-Alkyl, $C_1$-$C_8$-Alkoxy, -CO-$R_5$ oder -$NR_6R_7$,
$R_3$ und $R_4$ unabhängig voneinander für Wasserstoff oder Methyl,
$R_5$ für $C_1$-$C_8$-Alkoxy oder -$NR_8R_9$,
$R_6$ für $C_1$-$C_4$-Alkyl,
$R_7$ für $C_1$-$C_4$-Alkyl oder
$R_6$ und $R_7$ zusammen für eine $C_4$-$C_6$-Alkylenkette, welche durch Sauerstoff, Schwefel oder -$NCH_3$- unterbrochen sein kann,
$R_8$ für Wasserstoff oder $C_1$-$C_4$-Alkyl und
$R_9$ für Wasserstoff, $C_1$ -$C_4$-Alkyl, Benzyl, Phenyl oder durch Halogen oder Methyl substituiertes Phenyl oder
$R_8$ und $R_9$ zusammen für eine $C_4$-$C_6$-Alkylenkette, welche durch Sauerstoff, Schwefel oder -$NCH_3$- unterbrochen sein kann, stehen, zusammen mit geeigneten Trägern und/oder Zuschlagstoffen.

2. Mittel gemäss Anspruch 1, dadurch gekennzeichnet, dass $R_1$ für $C_1$-$C_4$-Alkyl, $R_2$ für $C_1$-$C_4$-Alkyl, $C_1$-$C_4$-Alkoxy, $C_1$-$C_4$-Alkoxycarbonyl, Di-($C_1$-$C_4$-alkyl)-amino oder -$CONR_8R_9$ stehen, wobei $R_8$ $C_1$-$C_4$-Alkyl, $R_9$ Wasserstoff, $C_1$-$C_4$-Alkyl oder $R_8$ und $R_9$ zusammen eine $C_1$-$C_6$-Alkylenkette, welche durch Sauerstoff unterbrochen sein kann, bedeuten.

3. Mittel gemäss Anspruch 2, dadurch gekennzeichnet, dass $R_2$ für $C_1$-$C_4$-Alkyl, $C_1$-$C_4$-Alkoxy, $C_1$-$C_4$-Alkoxycarbonyl, Di-($C_1$-$C_4$-alkyl)-aminocarbonyl oder $C_4$-$C_6$-Alkylenaminocarbonyl steht.

4. Mittel gemäss Anspruch 1, dadurch gekennzeichnet, dass $R_1$ für $C_1$-$C_4$-Alkyl, $R_2$ für $C_1$-$C_4$-Alkyl, $C_1$-$C_4$-Alkoxy, $C_1$-$C_4$-Alkoxycarbonyl oder Di-($C_1$-$C_2$-alkyl)-aminocarbonyl, $R_3$ für Methyl und $R_4$ für Wasserstoff stehen.

5. Mittel gemäss Anspruch 1 enthaltend einen Wirkstoff ausgewählt aus der Gruppe

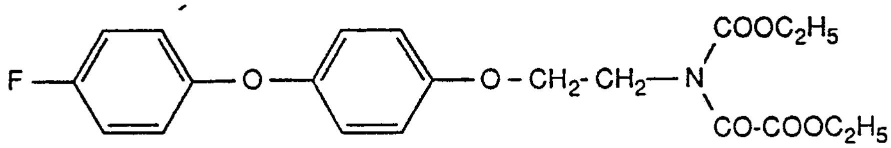
,

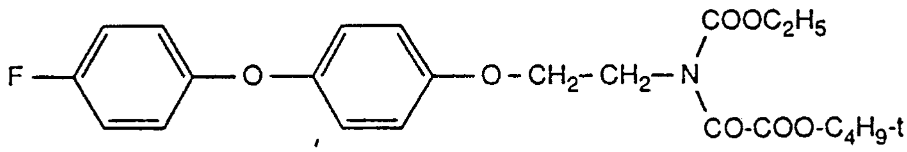
,

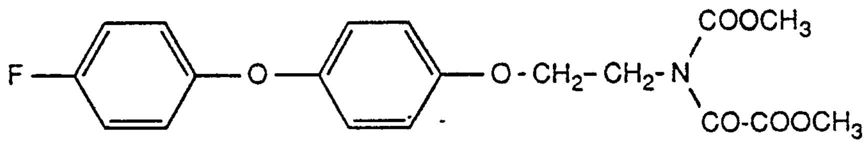
,

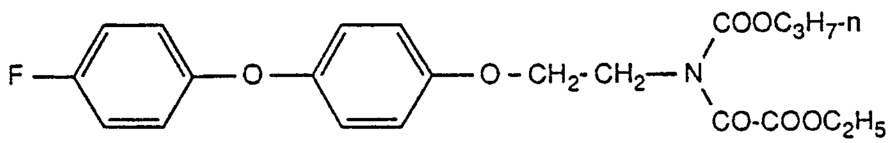
,

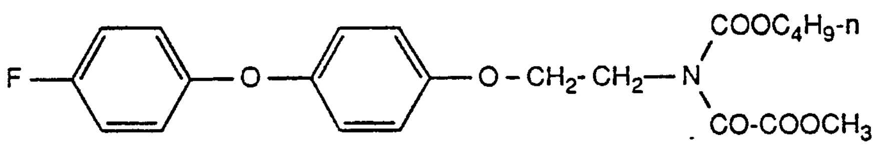
,

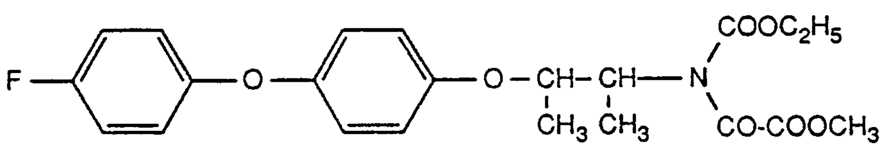
,

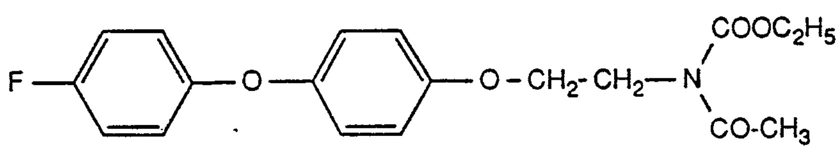
,

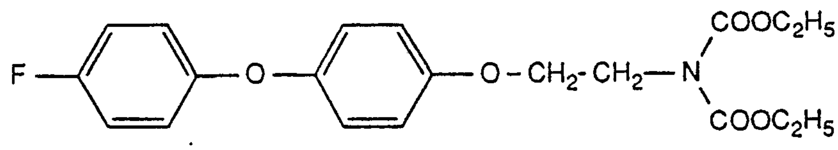

,

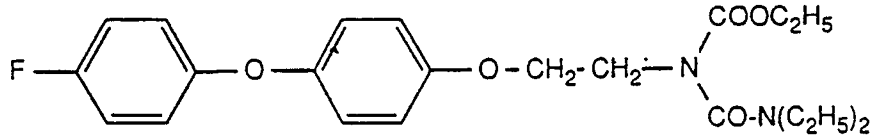

,

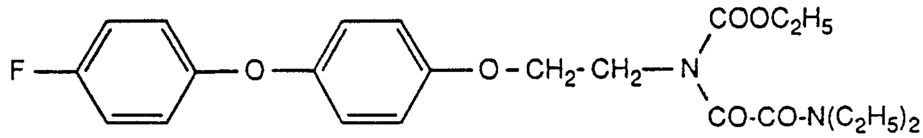

,

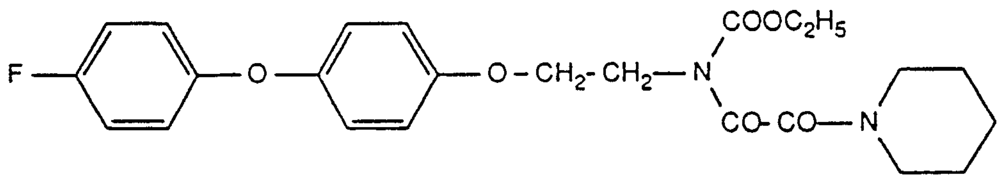

,

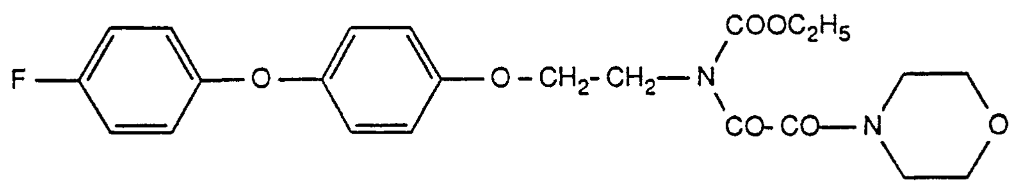

,

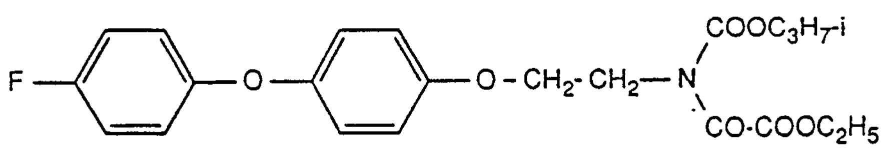

,

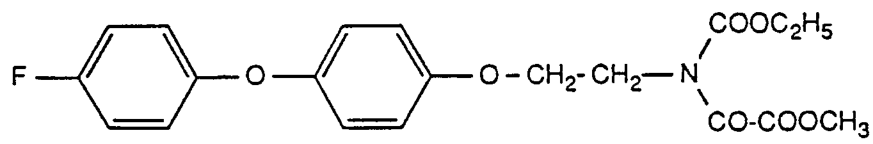

und

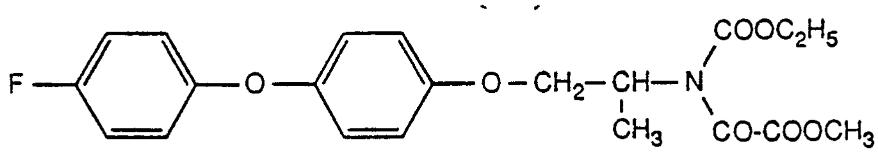

.

6. Verfahren zur Herstellung der Verbindungen der Formel I, dadurch gekennzeichnet, dass man einen Fluorphenoxyphenoxyäthylcarbaminsäureester der Formel II

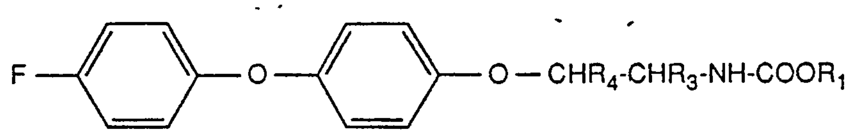

(II)

worin $R_1$, $R_3$ und $R_4$ die unter Formel I angegebenen Bedeutungen haben, mit einer Verbindung der Formel III

X-CO-$R_2$ (III)

worin $R_2$ die unter Formel I angegebene Bedeutung hat, und X für Chlor, Brom, -O-CO-$R_2$ oder -O-CO($C_1$-$C_4$-Alkyl) steht, acyliert.

7. Verfahren gemäss Anspruch 6, dadurch gekennzeichnet, dass man die Acylierung in Gegenwart einer Base durchführt.

8. Verwendung einer Verbindung der Formel I

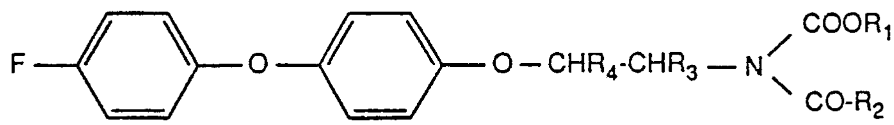

(I),

worin

$R_1$ für $C_1$-$C_8$-Alkyl, $C_3$-$C_4$-Alkenyl oder $C_3$-$C_4$-Alkinyl,
$R_2$ für $C_1$-$C_8$-Alkyl, $C_1$-$C_8$-Alkoxy, -CO-$R_5$ oder -$NR_6R_7$,
$R_3$ und $R_4$ unabhängig voneinander für Wasserstoff oder Methyl,
$R_5$ für $C_1$-$C_8$-Alkoxy oder -$NR_8R_9$,
$R_6$ für $C_1$-$C_4$-Alkyl,
$R_7$ für $C_1$-$C_4$-Alkyl oder
$R_6$ und $R_7$ zusammen für eine $C_4$-$C_6$-Alkylenkette, welche durch Sauerstoff, Schwefel oder -$NCH_3$- unterbrochen sein kann,
$R_8$ für Wasserstoff oder $C_1$-$C_4$-Alkyl und
$R_9$ für Wasserstoff, $C_1$-$C_4$-Alkyl, Benzyl, Phenyl oder durch Halogen oder Methyl substituiertes Phenyl oder
$R_8$ und $R_9$ zusammen für eine $C_4$-$C_6$-Alkylenkette, welche durch Sauerstoff, Schwefel oder -$NCH_3$- unterbrochen sein kann, stehen, zur Bekämpfung von Schädlingen.

9. Verwendung gemäss Anspruch 8 zur Bekämpfung von Arthropoden, insbesondere von Insekten und Arachniden.

10. Verwendung gemäss Anspruch 9 zur Bekämpfung von phytophagen Schadinsekten und -milben einschliesslich ihrer Larven und Eier.

11. Verwendung gemäss Anspruch 9 zur Chemosterilisierung pflanzenschädigender Schadinsekten und -milben.

12. Verfahren zur Herstellung der Verbindungen IVa

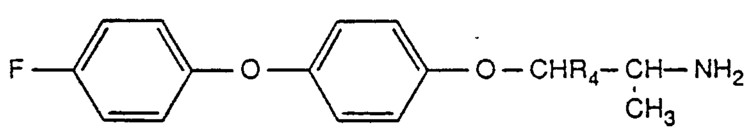

(IVa)

und IVb

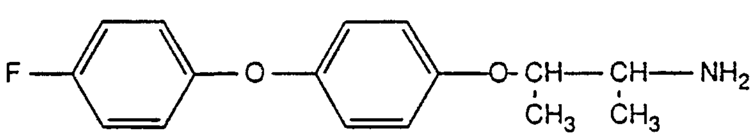

(IVb),

dadurch gekennzeichnet, dass man 4-(4-Fluorphenoxy)-phenol mit einer Verbindung der Formel Hal-$CHR_4$-

$CO\text{-}CH_3$, worin $R_4$ Wasserstoff oder Methyl und Hal Cl oder Br bedeuten, in Gegenwart eines Alkalicarbonats erhitzt, das erhaltene 4-(4-Fluorphenoxy)-phenoxy-aceton der Formel

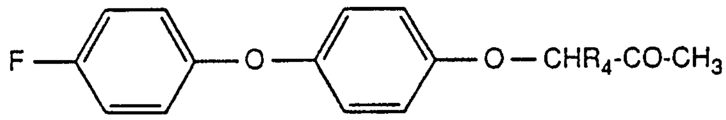

worin $R_4$ Wasserstoff oder Methyl bedeutet, in Gegenwart von einem Hydrierungskatalysator, mit Ammoniak und Wasserstoff unter Druck behandelt.

13. Verfahren zur Herstellung der Verbindungen der Formel I, worin $R_2$ für die Gruppe $-CO-R_5$ steht, dadurch gekennzeichnet, dass man die Verbindung der Formel II nach Anspruch 6 zunächst in einem inerten Lösungsmittel mit Oxalylchlorid umsetzt, und das entstehende Zwischenprodukt der Formel

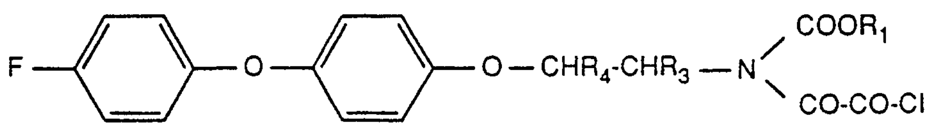

worin $R_1$, $R_3$ und $R_4$ die unter Formel I gegebenen Bedeutungen haben, in Gegenwart einer Base mit einem Alkohol oder einem Amin der Formel

$H\text{-}R_5$

worin $R_5$ die unter Formel I gegebene Bedeutung hat, umsetzt.